# EUROPEAN PATENT APPLICATION

(11) **EP 2 110 661 A1**
(43) Date of publication of application: **21.10.2009**
(21) Application number: 08710690.2
(22) Date of filing: 31.01.2008
(51) Int. Cl.: G01N 27/406, G01N 27/416, H01M 8/04, H01M 8/10

(54) **HYDROGEN GAS SENSOR**

(30) Priority: 02.02.2007 JP 2007023799; 07.03.2007 JP 2007056675; 06.08.2007 JP 2007204625; 29.08.2007 JP 2007222682
(71) Applicant: Gunze Limited, Ayabe-shi, Kyoto 623-8511 (JP)
(72) Inventor: HANE, Tomoko, Moriyama-shi Shiga 524-8501 (JP); KIYOHARA, Akio, Moriyama-shi Shiga 524-8501 (JP)
(74) Representative: Betten & Resch
(86) International application number: PCT/JP2008/051592
(87) International publication number: WO 2008/093813

(57) **Abstract**

Disclosed herein is a hydrogen gas sensor which requires no purging, has excellent hydrogen gas selectivity, and can be produced at relatively low cost. The hydrogen gas sensor includes a detection unit having a base body composed of a solid polymer electrolyte or a carbon-containing solid polymer electrolyte obtained by dispersing a carbon material in a solid polymer electrolyte and a catalyst layer provided on one of the surfaces of the base body to perform catalytic function on contact with hydrogen gas.

## Description

### TECHNICAL FIELD

The present invention relates to a hydrogen gas sensor for detecting a hydrogen gas concentration in a gas phase or a liquid phase and a hydrogen gas concentration in a gas to be supplied to a hydrogen storage alloy.

### BACKGROUND ART

A hydrogen gas sensor capable of accurately detecting a hydrogen concentration is very much needed for hydrogen energy systems represented by fuel cells.

Patent Document 1 proposes a hydrogen gas sensor which can measure a hydrogen concentration in a gas phase or a liquid phase without heating a sensitive part to high temperature and which achieves prevention of deterioration of a diaphragm and an electrolytic solution and reduction in size and weight by eliminating the need to use a diaphragm and an electrolytic solution.

The hydrogen gas sensor described in Patent Document 1 has a sensitive part configured to cause a change in electric resistance on contact with hydrogen. The sensitive part is made of a hydrogen storage simple metal or alloy or a material selected from nano-carbon materials, hydrogen storage simple metals, and hydrogen storage alloys.

Further, Patent Document 2 proposes a hydrogen gas sensor having a sensitive part made of a metal oxide semiconductor such as TiO₂, SrTiO₃, or BaTiO₃ and a pair of electrodes provided on the sensitive part to measure electric resistance.

Further, Patent Document 3 proposes a device for measuring the amount of permeated hydrogen gas, which has a fuel battery cell-type hydrogen measuring sensor element, a hollow cylindrical body, and a non-resistance ammeter. The sensor element is obtained by attaching a carbon cloth electrode to each of the both surfaces of a solid polymer electrolyte membrane. The hollow cylindrical body has two openings, and the edge of one of the openings abuts against the surface of an object to be measured. The sensor element is placed in the hollow cylindrical body so as to be substantially parallel to the plane of the one of the openings so that the interior of the hollow cylindrical body is partitioned by the sensor element. The non-resistance ammeter is provided to measure the value of electric current flowing between an anode and a cathode.
Patent Document 1: Japanese Patent Application Laid-open No. 2004-125513
Patent Document 2: Japanese Patent Application Laid-open No. 2002-071611
Patent Document 3: Japanese Patent Application Laid-open No. 2002-289243

### DISCLOSURE OF THE INVENTION

### PROBLEMS TO BE SOLVED BY THE INVENTION

However, since the hydrogen gas sensor described in Patent Document 1 is configured to detect a change in electric resistance caused by absorption of hydrogen gas into the sensitive part made of a hydrogen storage simple metal or alloy, absorbed hydrogen is kept stored in the sensitive part. Therefore, this hydrogen gas sensor has a problem that it cannot follow a dynamic change in a hydrogen gas concentration and requires purging to release absorbed hydrogen from the sensitive part to prepare for the next measurement.

Further, since the hydrogen gas sensor described in Patent Document 2 is configured to detect a change in the electric resistance of a metal oxide semiconductor caused by a reaction between hydrogen gas and oxygen adsorbed to the surface of the metal oxide semiconductor, the hydrogen gas sensor needs to have a heating element for increasing the temperature of the sensitive part to about 400°C to allow the adsorption and desorption of oxygen molecules to occur satisfactorily.

Therefore, the hydrogen gas sensor described in Patent Document 2 has a problem that it consumes a large amount of electric power and needs to have a heat-resistant structure. In addition, there is also a problem that this hydrogen gas sensor responds to not only hydrogen gas but also other flammable gases such as methane gas and carbon monoxide gas (i.e., has no gas selectivity).

Further, the device for measuring the amount of permeated hydrogen gas described in Patent Document 3 has a problem that a perfluorosulfonic acid resin membrane used as a solid polymer electrolyte membrane has good hydrogen ion conductivity under wet conditions but cannot effectively perform its function under dry conditions.

In addition, there is also a problem that the perfluorosulfonic acid resin membrane does not have a cross-linked structure and is therefore reduced in strength by containing a lot of water at high temperature. Further, the production cost of the perfluorosulfonic acid resin membrane is high due to a complicated production process. Therefore, from the viewpoint of cost reduction, there has been a demand for a further improved hydrogen gas sensor.

Meanwhile, a hydrogen gas sensor can be produced also by utilizing the power generation characteristics of an MEA (Membrane Electrode Assembly) generally used in fuel cells. However, since an MEA is produced by forming a catalyst electrode made of an expensive metal such as platinum on each of the both surfaces of a solid polymer electrolyte membrane, from the viewpoint of reduction in component cost, there has been a demand for a further improved hydrogen gas sensor.

Further, in such a hydrogen gas sensor using an MEA, once the MEA comes in contact with hydrogen gas, it keeps producing output for some time even after breaking off contact with hydrogen gas, and therefore, the output fall time after breaking off contact with hydrogen gas is long. From the viewpoint of detection of hydrogen gas, there has also been a demand for a hydrogen gas sensor having improved output fall characteristics.

In view of the above problems, it is an object of the present invention to provide a hydrogen gas sensor which requires no purging, has excellent hydrogen gas selectivity, and can be produced at relatively low cost.

### MEANS FOR SOLVING THE PROBLEMS

To achieve the above object, the hydrogen gas sensor of the present invention has a detection unit having a base body composed of a solid polymer electrolyte and a catalyst layer provided on one of surfaces of the base body.

The present inventors have already found that a detection unit having good responsivity to hydrogen gas can be obtained by forming a catalyst layer on one of the surfaces of a carbon base body composed of carbon paper, carbon cloth, carbon nanotubes, fullerene, graphite, or the like. However, such a detection unit has the drawback that sensitivity is low.

In order to overcome such a drawback, the present inventors have extensively studied, and as a result have found that a detection unit obtained by forming a catalyst layer made of a metal, which performs catalytic function on contact with hydrogen gas, or the like on one of the surfaces of a base body composed of a solid polymer electrolyte exhibits satisfactory power generation characteristics when hydrogen gas is supplied to the catalyst layer. In this case, unlike existing fuel cells, it is not necessary to provide a catalyst layer on both surfaces of a base body. Therefore, such a detection unit is advantageous in that it can be produced at low cost.

The base body is preferably composed of a carbon-containing solid polymer electrolyte in which carbon is dispersed, and a carbon-containing solid polymer electrolyte obtained by dispersing at least one carbon material selected from among carbon nanotubes, fullerene, graphite, nano-carbon, and carbon black in a solid polymer electrolyte can be suitably used.

As described above, when the base body is composed of only a solid polymer electrolyte, the hydrogen gas sensor has good sensitivity to hydrogen gas, but the output fall time is long. The present inventors have further extensively studied, and as a result have found that a hydrogen gas sensor excellent in both output rise characteristics and output fall characteristics and having satisfactory sensitivity to hydrogen gas can be obtained by using a base body composed of the above-described carbon-containing solid polymer electrolyte.

However, in a case where the carbon material to be dispersed in the above-described carbon-containing solid polymer electrolyte constituting the base body is selected from the group consisting of fullerene, nano-diamond, and nanoporous carbon, the hydrogen gas sensor has sensitivity to a gas having a very low hydrogen gas concentration, but tends to be saturated when a gas having a relatively high hydrogen gas concentration is supplied, and is therefore less likely to accurately detect a hydrogen gas concentration. Further, in this case, the hydrogen gas sensor also tends to fluctuate in output (i.e., tends to show low output stability) in the early stage in the absence of hydrogen gas.

On the other hand, in a case where the carbon material is selected from the group consisting of carbon nanotubes, graphite, nano-carbon, and carbon black, the output stability of the hydrogen gas sensor in the absence of hydrogen gas can be ensured, but the hydrogen gas sensor tends to have sensitivity only to a gas having a relatively high hydrogen gas concentration.

However, when a carbon-containing solid polymer electrolyte obtained by mixing a solid polymer electrolyte with at least one carbon material selected from the group consisting of carbon black, graphite, nano-carbon, and carbon nanotubes and at least one carbon material selected from the group consisting of fullerene, nano-diamond, and nanoporous carbon is used as the base body, it is possible to ensure the output stability of the hydrogen gas sensor in the absence of hydrogen gas while properly adjusting the sensitivity to hydrogen gas.

That is, a carbon-containing solid polymer electrolyte obtained by mixing a solid polymer electrolyte with at least one carbon material selected from the group consisting of carbon black, graphite, nano-carbon, and carbon nanotubes and at least one carbon material selected from the group consisting of fullerene, nano-diamond, and nanoporous carbon can be suitably used as the base body.

It is preferred that a ratio of the at least one carbon material selected from the group consisting of fullerene, nano-diamond, and nanoporous carbon with respect to total carbon materials contained in the base body is adjusted according to a target range of a hydrogen gas concentration to be detected, and it is possible to properly adjust the sensitivity to hydrogen gas by adjusting the ratio of the at least one carbon material as described above.

When a percentage by weight X of the at least one carbon material selected from the group consisting of fullerene, nano-diamond, and nanoporous carbon with respect to total carbon materials contained in the base body is 50 or more but less than 100 (50 ≤ X < 100), the output is relatively stabilized even in the absence of hydrogen gas and a very low hydrogen gas concentration can be detected at high sensitivity.

When a percentage by weight X of the at least one carbon material selected from the group consisting of fullerene, nano-diamond, and nanoporous carbon with respect to total carbon materials contained in the base body is more than 0 but less than 50 (0 < X < 50), the output is stabilized in the absence of hydrogen gas and a high hydrogen gas concentration can be properly detected.

It is preferred that the carbon-containing solid polymer electrolyte is formed by dispersing the carbon material in a solution of a solid polymer electrolyte to obtain a dispersed solution, molding the dispersed solution into a predetermined shape to obtain a molded product, and thermally drying the molded product.

Additionally, it is preferred that the hydrogen gas sensor has a detection unit having a base body composed of a carbon-containing solid polymer electrolyte obtained by mixing a solution of a solid polymer electrolyte with two types of carbon materials different in dispersibility in a solvent contained in the solution of the solid polymer electrolyte and a catalyst layer provided on one of surfaces of the base body. Such a configuration also enables proper adjustment of the sensitivity to hydrogen gas depending on the combination of carbon materials.

It is preferred that the catalyst layer is made of a metal or an alloy which performs catalytic function on contact with hydrogen gas or an organic metal or an organic substance which exhibits catalytic activity on contact with hydrogen gas.

Further, it is also preferred that the catalyst layer is made of a material containing molybdenum carbide which performs catalytic function on contact with hydrogen gas. This is because molybdenum carbide is very cheap in spite of the fact that its catalytic performance is comparable to that of platinum. This leads to a significant reduction in the production cost of the hydrogen gas sensor.

Even if the catalytic performance of Molybdenum carbide is regarded as being slightly lower than that of platinum, since molybdenum carbide is cheap, an increase in the amount of molybdenum carbide used as a catalyst can be readily contemplated to cover the gap in catalytic performance.

It is preferred that the catalyst layer is formed by allowing a catalyst to be supported on one of surfaces of the base body by a thin film forming method selected from among a sputtering method, an ion beam method, a coating method, and a vapor deposition method. Such a thin film forming method enables control of the particle size distribution and the amount of the catalyst supported on the support to a suitable value.

Further, the detection unit having a base body and a catalyst layer provided on one of the surfaces of the base body is preferably bonded to gas-permeable electrodes. This makes it possible, when hydrogen gas is supplied to the catalyst layer-side electrode and oxygen or air is supplied to the opposite electrode, to properly take out a generated voltage to the outside.

### EFFECT OF THE INVENTION

As has been described above, according to the present invention, it is possible to provide a hydrogen gas sensor which requires no purging, has excellent hydrogen gas selectivity, and can be produced at relatively low cost while achieving low power consumption and high responsivity and sensitivity.

### BRIEF DESCRIPTION OF THE DRAWINGS

Fig. 1 is a diagram showing the structure of a hydrogen gas sensor using a solid polymer electrolyte as a base body.
Fig. 2 is a diagram showing the structure of a hydrogen gas sensor having a gas diffusion layer provided on each electrode.
Fig. 3 is a diagram showing the structure of prototype hydrogen gas sensors obtained in Example 1.
Fig. 4A is a graph showing the hydrogen gas detection characteristics of a hydrogen gas sensor obtained in Example 1-1.
Fig. 4B is a graph showing the hydrogen gas concentration-voltage characteristics of the hydrogen gas sensor obtained in Example 1-1.
Fig. 5A is a graph showing the hydrogen gas detection characteristics of a hydrogen gas sensor obtained in Example 1-2.
Fig. 5B is a graph showing the hydrogen gas concentration-voltage characteristics of the hydrogen gas sensor obtained in Example 1-2.
Fig. 6A is a graph showing the hydrogen gas detection characteristics of a hydrogen gas sensor obtained in Example 1-4.
Fig. 6B is a graph showing the hydrogen gas concentration-voltage characteristics of the hydrogen gas sensor obtained in Example 1-4.
Fig. 7A is a graph showing the hydrogen gas detection characteristics of a hydrogen gas sensor obtained in Example 1-5.
Fig. 7B is a graph showing the hydrogen gas concentration-voltage characteristics of the hydrogen gas sensor obtained in Example 1-5.
Fig. 8A is a graph showing the hydrogen gas detection characteristics of a hydrogen gas sensor obtained in Example 1-6.
Fig. 8B is a graph showing the hydrogen gas concentration-voltage characteristics of the hydrogen gas sensor obtained in Example 1-6.
Fig. 9 is a diagram showing the structure of a hydrogen gas sensor using a base body composed of a carbon-containing solid polymer electrolyte.
Fig. 10 is a diagram showing the structure of prototype hydrogen gas sensors obtained in Example 2.
Fig. 11A is a graph showing the hydrogen gas detection characteristics of a hydrogen gas sensor obtained in Example 2-1 using a carbon material (A).
Fig. 11B is a graph showing the hydrogen gas detection characteristics of a hydrogen gas sensor obtained in Example 2-1 using a carbon material (B).
Fig. 11C is a graph showing the hydrogen gas detection characteristics of a hydrogen gas sensor obtained in Example 2-1 using a carbon material (C).
Fig. 12A is a graph showing the hydrogen gas detection characteristics of a hydrogen gas sensor obtained in Example 2-1 using a carbon material (D).
Fig. 12B is a graph showing the hydrogen gas detection characteristics of a hydrogen gas sensor obtained in Example 2-1 using a carbon material (E).
Fig. 12C is a graph showing the hydrogen gas detection characteristics of a hydrogen gas sensor obtained in Example 2-2.
Fig. 13 is a scanning electron micrograph of a base body used in the hydrogen gas sensor obtained in Example 2-1.

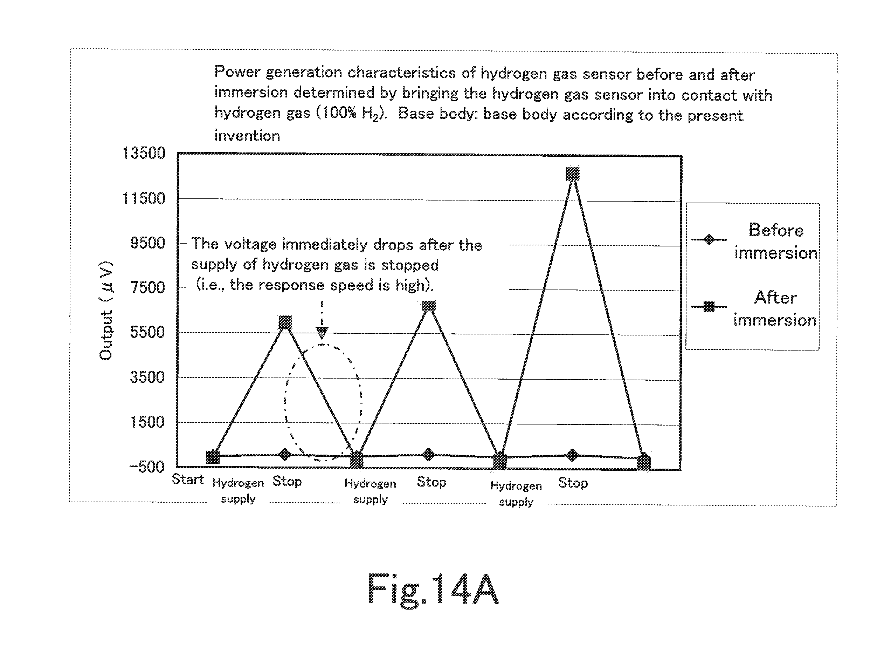

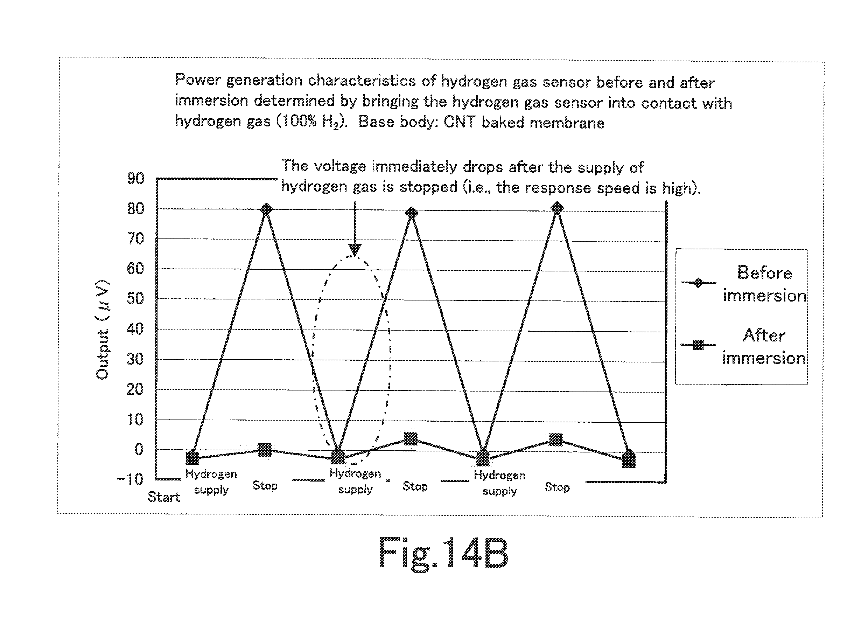

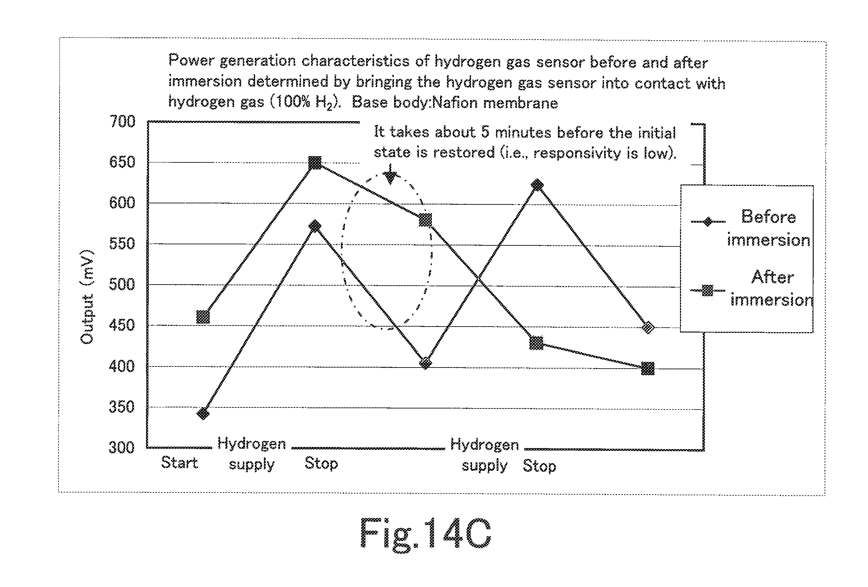

Figs. 14A to 14C are graphs each showing the power generation characteristics of a hydrogen gas sensor obtained in Example 2-3 before and after immersion of a base body in water, wherein Fig. 14A is a graph showing the hydrogen gas detection characteristics of a hydrogen gas sensor using a base body according to the present invention, Fig. 14B is a graph showing the hydrogen gas detection characteristics of a hydrogen gas sensor using a baked membrane composed of carbon nanotubes as a base body, and Fig. 14C is a graph showing the hydrogen gas detection characteristics of a hydrogen gas sensor using a Nafion membrane as a base body.
Fig. 15A is a graph for explaining the hydrogen gas detection characteristics of a hydrogen gas sensor using a carbon base body.
Fig. 15B is a graph for explaining the hydrogen gas detection characteristics of a hydrogen gas sensor using a solid polymer electrolyte as a base body.
Fig. 16 is a diagram showing the structure of a hydrogen gas sensor using a base body composed of a carbon-containing solid polymer electrolyte.
Fig. 17 is a diagram showing the structure of a hydrogen gas sensor using a base body composed of a carbon-containing solid polymer electrolyte containing a mixture of two types of carbon materials different in specific gravity.
Fig. 18 is a diagram showing the structure of prototype hydrogen gas sensors obtained in Example 3.
Fig. 19A is a table showing the characteristics of a hydrogen gas sensor obtained in Example 3-1.
Fig. 19B is a graph showing the output characteristics of the hydrogen gas sensor obtained in Example 3-1.
Fig. 20A is a table showing the characteristics of a hydrogen gas sensor obtained in Example 3-2.
Fig. 20B is a graph showing the output characteristics of the hydrogen gas sensor obtained in Example 3-2.
Fig. 21A is a table showing the characteristics of a hydrogen gas sensor obtained in Example 3-3.
Fig. 21B is a graph showing the output characteristics of the hydrogen gas sensor obtained in Example 3-3.
Fig. 22A is a graph showing the output characteristics of a hydrogen gas sensor using a base body composed of a solid polymer electrolyte in which only fullerene is dispersed.
Fig. 22B is a graph showing the output characteristics of a hydrogen gas sensor using a base body composed of a solid polymer electrolyte in which only carbon nanotubes are dispersed.
Fig. 23 is a diagram showing the structure of a hydrogen gas sensor using a solid polymer electrolyte as a base body and molybdenum carbide as a catalyst.
Fig. 24 is a diagram showing the structure of a hydrogen gas sensor having a gas diffusion layer provided on each electrode.
Fig. 25 is a diagram showing the structure of a hydrogen gas sensor having a stack structure.
Fig. 26 is a diagram for explaining the structure of a hydrogen gas sensor system having a control unit and fans.
Fig. 27 is a block diagram showing the functions of a sensor, a control unit, and the like of the hydrogen gas sensor system.
Fig. 28 is a control flow chart of the hydrogen gas sensor system.
Fig. 29 is a diagram showing the structure of a hydrogen gas sensor having a base body and catalyst layers provided on both surfaces of the base body.

### DESCRIPTION OF REFERENCE NUMERALS

- 1: hydrogen gas sensor
- 2: base body (solid polymer electrolyte)
- 3: catalyst layer
- 3a: catalyst
- 4: electrode
- 4a: anode electrode
- 4b: cathode electrode
- 5: acrylic resin plate
- 5a: opening provided in the plate 5
- 6: voltage detection circuit
- 7: gas diffusion layer
- 22: base body (carbon-containing solid polymer electrolyte)
- 23: base body (carbon-containing solid polymer electrolyte)
- 40: electrode pair
- 40a,: 40b, 40c electrode plate
- 50a,: 50b gas diffusion layer
- 60a,: 60b acrylic plate
- 100: control unit
- 101: CPU
- 102: memory
- 105a, 105b: fan
- 200: base body
- 300: Mo₂C catalyst particle
- C: carbon
- C1: graphite, carbon nanotubes
- C2: fullerene, nano-diamond
- S: solid polymer electrolyte

### BEST MODES FOR CARRYING OUT THE INVENTION

Hereinbelow, a hydrogen gas sensor according to the present invention will be described. The hydrogen gas sensor includes a detection unit having a base body composed of a solid polymer electrolyte and a catalyst layer provided on one of the surfaces of the base body, and the detection unit is bonded to gas-permeable electrodes.

As the base body, one composed of a solid polymer electrolyte or one composed of a solid polymer electrolyte mixed with a carbon material can be used. Each of such base bodies will be described below.

### (Embodiment 1: Structure Using Base Body Composed of Solid Polymer Electrolyte)

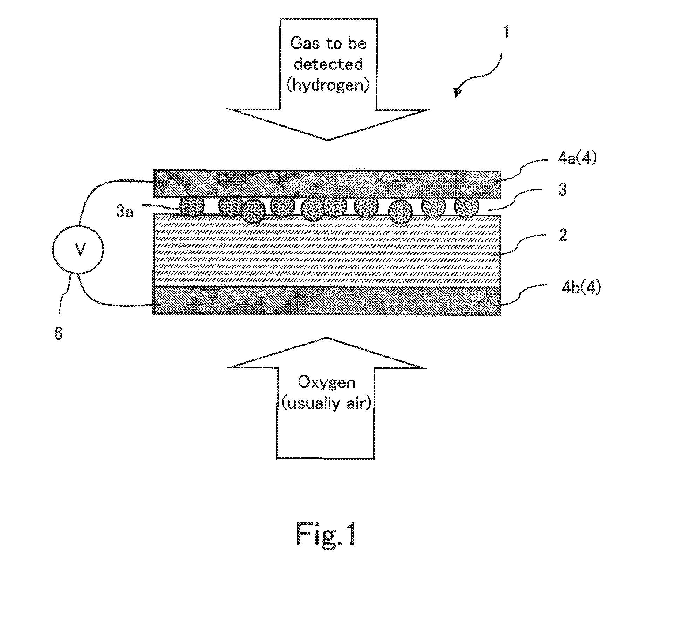

As shown in Fig. 1, a hydrogen gas sensor 1 has a base body 2 composed of a solid polymer electrolyte, a catalyst layer 3 which performs catalytic function on contact with hydrogen gas, and gas-permeable electrodes 4. The catalyst layer 3 is provided on one of the surfaces of the base body 2, and each of the both surfaces of the base body 2 is bonded to the electrode 4.

The solid polymer electrolyte constituting the base body 2 is not particularly limited as long as it has proton conductivity. Preferred examples of such a solid polymer electrolyte include solid polymer electrolyte membranes composed of perfluoro-based polymers having high proton conductivity such as perfluorosulfonic acid-based polymers and perfluorocarboxylic acid-based polymers or partially sulfonated styrene-olefin copolymers such as Poly(styrene-ran-ethylene), sulfonated polymer (polystyrene-based sulfonic acid resin). More specifically, NAFION (registered trademark of Du Pont) and ACIPLEX (registered trademark of Asahi Kasei Corporation) are preferably used.

The catalyst layer 3 is composed of a catalyst 3a. Preferred examples of the catalyst 3a include metals and alloys which perform catalytic function on contact with hydrogen gas and organic metals and organic substances which exhibit catalytic activity on contact with hydrogen gas.

The catalyst 3a is allowed to be supported on at least one of the surfaces of the base body 2 by a thin film forming method using sputtering.

As the catalyst 3a, platinum (Pt) having high catalytic activity or a platinum alloy is preferably used. However, at least one metal selected from among gold (Au), silver (Ag), iridium (Ir), palladium (Pd), ruthenium (Ru), osmium (Os), nickel (Ni), tungsten (W), molybdenum (Mo), manganese (Mn), yttrium (Y), vanadium (V), niobium (Nb), titanium (Ti), zirconia (Zr), and rare-earth metals or an alloy of two or more of these metals may also be used as the catalyst 3a.

Further, as described above, an organic metal or an organic substance which exhibits catalytic activity on contact with hydrogen gas may also be used as the catalyst 3a. Examples of such an organic metal catalyst include N,N'-Bis(salicylidene)ethylene-diamino-metal (=Ni, Fe, V or the like) and N,N'-mono-8-quinolyl-σ-phenylenediamino-metal (=Ni, Fe, V or the like). Examples of such an organic substance include pyrrolo-pyrrole red pigment and dipyridyl derivatives.

Further, molybdenum carbide (Mo₂C) which performs catalytic function on contact with hydrogen gas or a material containing molybdenum carbide (Mo₂C) may also be used as the catalyst 3a. It is to be noted that an embodiment using molybdenum carbide (No₂C) as a catalyst will be described later in detail.

In a case where sputtering is employed to allow the catalyst 3a to be supported on one of the surfaces of the base body 2, the sputtering time is preferably shorter than 90 seconds, more preferably 60 seconds or shorter. The DC or RF power during sputtering is not particularly limited, but is preferably 1.2 W/cm² or more.

The electrode 4 can be constituted from, for example, a copper-nickel alloy thin film having a plurality of pores. Alternatively, the electrode 4 may also be constituted from a porous metal sintered body having good electric conductivity.

When hydrogen gas flows into an electrode 4a of the hydrogen gas sensor 1, hydrogen splits into protons and electrons, and then the electrons are supplied to an external circuit connected to the electrode 4a and the protons are transferred through the base body 2 to an electrode 4b opposed to the electrode 4a so that output is taken out, that is, hydrogen gas is detected. At this time, a hydrogen gas concentration can be measured by detecting a current value or a voltage value having a correlation with a hydrogen gas concentration using the external circuit.

More specifically, a hydrogen gas concentration in an analyte gas can be measured by positioning the hydrogen gas sensor 1 so that the electrode 4a side thereof having at least the catalyst layer 3 is exposed to the analyte gas and the other electrode 4b is brought into contact with outside air or oxygen gas and by providing a voltage detection circuit 6 between these electrodes 4a and 4b.

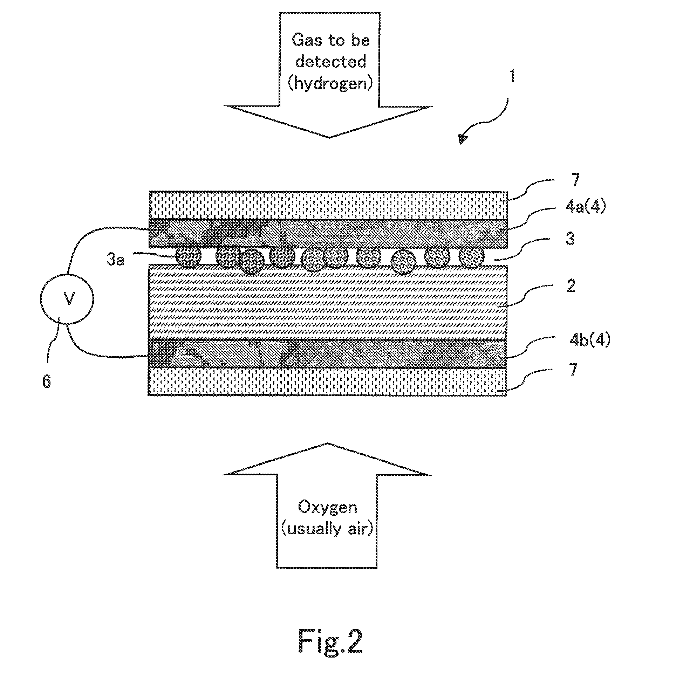

As shown in Fig. 2, a gas diffusion layer 7 constituted from carbon paper, carbon cloth, or the like may be provided on the surface of each of the electrodes 4a and 4b so that an analyte gas is uniformly supplied to the electrode 4a and oxygen gas or air is uniformly supplied to the electrode 4b.

The hydrogen gas sensor 1 may further have a gas sending system, such as a fan, for supplying an analyte gas to the electrode 4a side having the catalyst layer 3 and continuously or intermittently supplying oxygen gas or air to the electrode 4b opposed to the electrode 4a.

In this case, a gas sending system for supplying an analyte gas to the electrode 4a side and a gas sending system for supplying air to the electrode 4b opposed, to the electrode 4a may be provided separately. These gas sending systems may be alternately operated.

More specifically, the hydrogen gas sensor 1 may further have a control unit for controlling these gas sending systems to alternately perform the operation of supplying an analyte gas to the electrode 4a side of the hydrogen gas sensor 1 by the use of one of the gas sending systems and then stopping the one of the gas sending systems to stop the supply of the analyte gas and the operation of supplying air to the electrode 4b side of the hydrogen gas sensor 1 by the use of the other gas sending system and then stopping the other gas sending system to stop the supply of the air. By providing such a control unit, it is possible not only to detect a hydrogen gas concentration but also to backwash the hydrogen gas sensor 1 to remove dust attached to the electrodes 4a and 4b and the gas diffusion layers 7 and residual gas in the gas diffusion layers 7.

The first embodiment of the hydrogen gas sensor has been described with reference to a case where the catalyst layer 3 is directly formed on the base body 2, but is not limited to this case. For example, a coating layer may be formed by coating one of the surfaces of the base body 2 with carbon fibers or carbon nanotubes to allow a catalyst to be supported on the surface of the coating layer. The coating layer formed from carbon nanotubes is excellent as a catalyst support because it has a large specific surface area.

Further, the electrode 4 of the hydrogen gas sensor 1 is not limited to one made of a metal that is a good electric conductor, and may be formed from carbon paper or carbon cloth having high electric conductivity and gas permeability. In this case, the surface of the electrode can function as a catalyst support.

Further, the first embodiment of the hydrogen gas sensor has been described with reference to a case where sputtering is employed to form the catalyst layer 3 on the base body 2, but a well-known method other than sputtering may be employed. Examples of such a well-known method include an ion beam method, a vacuum vapor deposition method, an electron irradiation method, CVD, PVD, impregnation, spray coating, spray thermal decomposition, mixing, spraying, coating using rollers or applicators, screen printing, a kneading method, photoelectrolysis, a coating method, a sol-gel method, a dipping method, an ink-jet method, and a noble metal complex reduction method.

Further, the first embodiment of the hydrogen gas sensor has been described with reference to a case where the base body 2 has a film shape, but the thickness of the base body 2 is not particularly limited and can be appropriately set as long as the desired effect of the present invention can be obtained. Further, the base body is not limited to a sheet-shaped one and may be a three-dimensional one. In this case, the electrodes are not always provided on the surfaces of the base body opposed to each other.

### <Example 1: Structure Using Solid Polymer Electrolyte as Base Body> Example 1-1

A perfluoro-based polymer membrane having a thickness of 100 µm (Nafion NRE-212 membrane available from Du Pont) was prepared as a base body 2 composed of a solid polymer electrolyte. A piece measuring 5 cm square was cut from the Nafion membrane, and was sandwiched between two sheets of filter paper and pressed from above with an iron heated to 130°C to remove water. The piece of Nafion membrane was repeatedly heated using the iron until no water could penetrate through the filter paper.

The piece of Nafion membrane, from which water had been fully removed in such a manner as described above, was subjected to sputtering for 30 seconds to allow platinum (Pt) to be supported as a catalyst on one of the surfaces thereof in an area measuring 4 cm square. In this way, a catalyst layer 3 having a thickness of 20 nm was formed. The sputtering of platinum (Pt) was performed under the conditions where the RF power was 300 W and the flow rate of argon (Ar) gas was 20 cc/min.

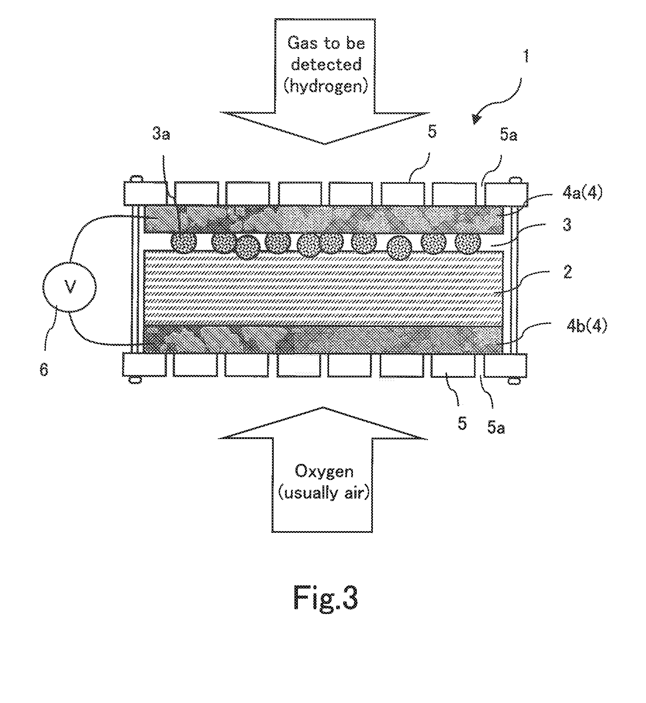

Then, as shown in Fig. 3, the Nafion membrane 2 having the catalyst layer 3 formed thereon was sandwiched between a pair of electrodes 4 made of copper nickel and having a plurality of pores, and the thus obtained structure was further sandwiched between two acrylic resin plates 5 having a plurality of openings 5a. The plates 5 were fixed to each other using bolts. In this way, a hydrogen gas sensor 1 was produced.

50 cc of hydrogen gas (100% H₂) and 50 cc of an air-based gas having a hydrogen gas concentration of 4% were each supplied with a syringe to the catalyst layer 3-side electrode 4a of the hydrogen gas sensor 1 at normal temperature and normal humidity (at 23°C and 55% RH), and a voltage was measured using a measuring instrument (MULTIMETER VOAC 7411 manufactured by Iwatsu Test Instrument Co.).

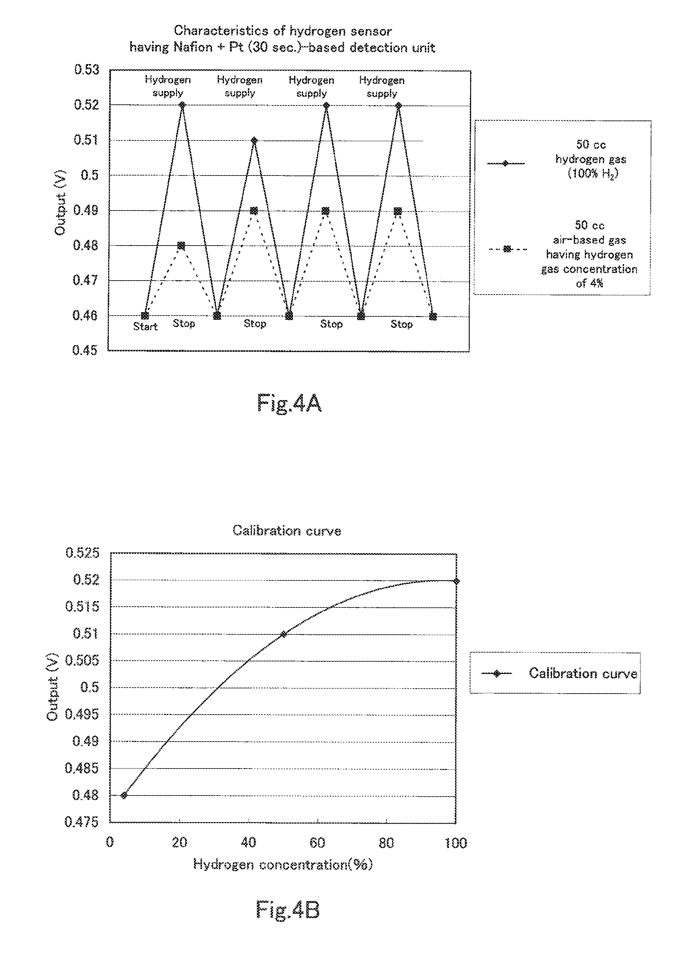

When each of the two types of gases was supplied for predetermined time and then the supply of the gas was stopped, a voltage increase of several hundreds of millivolts was measured. At this time, the hydrogen gas sensor of Example 1-1 exhibited the output characteristics shown in Fig. 4A and the hydrogen concentration-voltage characteristics shown in Fig. 4B.

### Example 1-2

A piece of Nafion membrane was prepared as a base body 2 composed of a solid polymer electrolyte in the same manner as in Example 1-1, and was then subjected to sputtering in the same manner as in Example 1-1 except that the sputtering time was changed to 10 seconds to allow platinum (Pt) to be supported as a catalyst on one of the surfaces thereof so that a catalyst layer 3 having a thickness of 4 nm was formed. In this way, a hydrogen gas sensor 1 having the same structure as shown in Fig. 3 was produced. The sputtering of platinum (Pt) was performed under the conditions where the RF power was 300 W and the flow rate of argon (Ar) gas was 20 cc/min.

50 cc of hydrogen gas (100% H₂) and 50 cc of an air-based gas having a hydrogen gas concentration of 4% were each supplied with a syringe to the catalyst layer 3-side electrode 4a of the hydrogen gas sensor 1 at normal temperature and normal humidity (at 23°C and 55% RH), and a voltage was measured using a measuring instrument (MULTIMETER VOAC 7411).

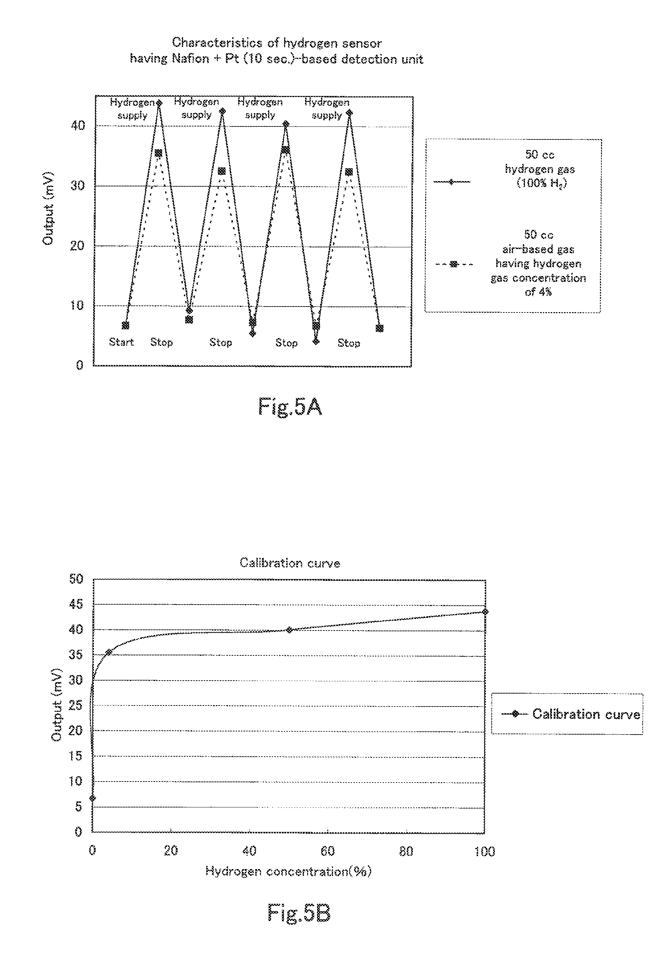

When each of the two types of gases was supplied for predetermined time and then the supply of the gas was stopped, a voltage increase of several tens of millivolts was measured. At this time, the hydrogen gas sensor of Example 1-2 exhibited the output characteristics shown in Fig. 5A and the hydrogen concentration-voltage characteristics shown in Fig. 5B.

### Example 1-3

A piece of Nafion membrane was prepared as a base body 2 composed of a solid polymer electrolyte in the same manner as in Example 1-1, and was then subjected to sputtering in the same manner as in Example 1-1 except that the sputtering time was changed to 30 seconds to allow platinum (Pt) to be supported as a catalyst on both surfaces thereof so that a catalyst layer 3 having a thickness of 20 nm was formed. In this way, a hydrogen gas sensor 1 having the same structure as shown in Fig. 3 was produced. The sputtering of platinum (Pt) was performed under the conditions where the RF power was 300 W and the flow rate of argon (Ar) gas was 20 cc/min.

50 cc of hydrogen gas (100% H₂) was supplied with a syringe to the catalyst layer 3-side electrode 4a of the hydrogen gas sensor 1 at normal temperature and normal humidity (at 23°C and 55% RH), and a voltage was measured using a measuring instrument (MULTIMETER VOAC 7411).

When the hydrogen gas was supplied for predetermined time and then the supply of the gas was stopped, a voltage of 780 mV was measured. It has been confirmed that a similar result can be obtained also when the catalyst layer is provided on each of the both surfaces of the Nation membrane.

### Example 1-4

A piece of Nafion membrane was prepared as a base body 2 composed of a solid polymer electrolyte in the same manner as in Example 1-1, and was then subjected to sputtering in the same manner as in Example 1-1 except that the sputtering time was changed to 30 seconds to allow palladium (Pd) to be supported as a catalyst on one of the surfaces thereof so that a catalyst layer 3 having a thickness of 20 nm was formed. In this way, a hydrogen gas sensor 1 having the same structure as shown in Fig. 3 was produced. The sputtering of palladium (Pd) was performed under the conditions where the RF power was 300 W and the flow rate of argon (Ar) gas was 20 cc/min.

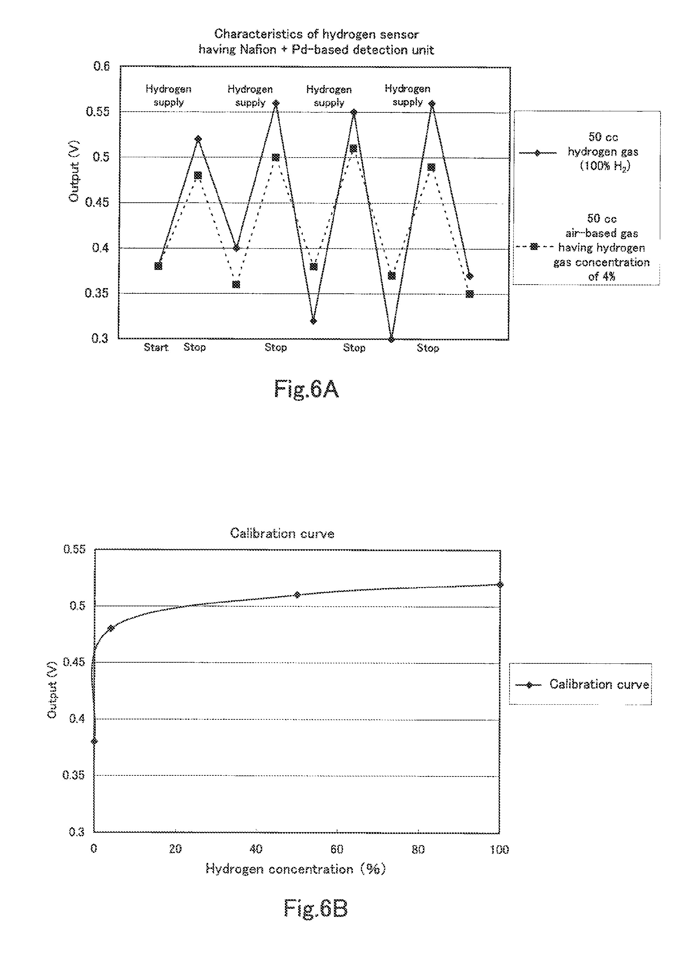

50 cc of hydrogen gas (100% H₂) and 50 cc of an air-based gas having a hydrogen gas concentration of 4% were each supplied with a syringe to the catalyst layer 3-side electrode 4a of the hydrogen gas sensor 1 at normal temperature and normal humidity (at 23°C and 55% RH), and a voltage was measured using a measuring instrument (MULTIMETER VOAC 7411). When each of the two types of gases was supplied for predetermined time and then the supply of the gas was stopped, a voltage increase of several hundreds of millivolts was measured. At this time, the hydrogen gas sensor of Example 1-4 exhibited the output characteristics shown in Fig. 6A and the hydrogen concentration-voltage characteristics shown in Fig. 6B.

### Example 1-5

A piece of Nation membrane was prepared as a base body 2 composed of a solid polymer electrolyte in the same manner as in Example 1-1, and was then subjected to sputtering in the same manner as in Example 1-1 except that the sputtering time was changed to 30 seconds to allow nickel (Ni) to be supported as a catalyst on one of the surfaces thereof so that a catalyst layer 3 having a thickness of 20 nm was formed. In this way, a hydrogen gas sensor 1 having the same structure as shown in Fig. 3 was produced. The sputtering of nickel (Ni) was performed under the conditions where the RF power was 300 W and the flow rate of argon (Ar) gas was 20 cc/min.

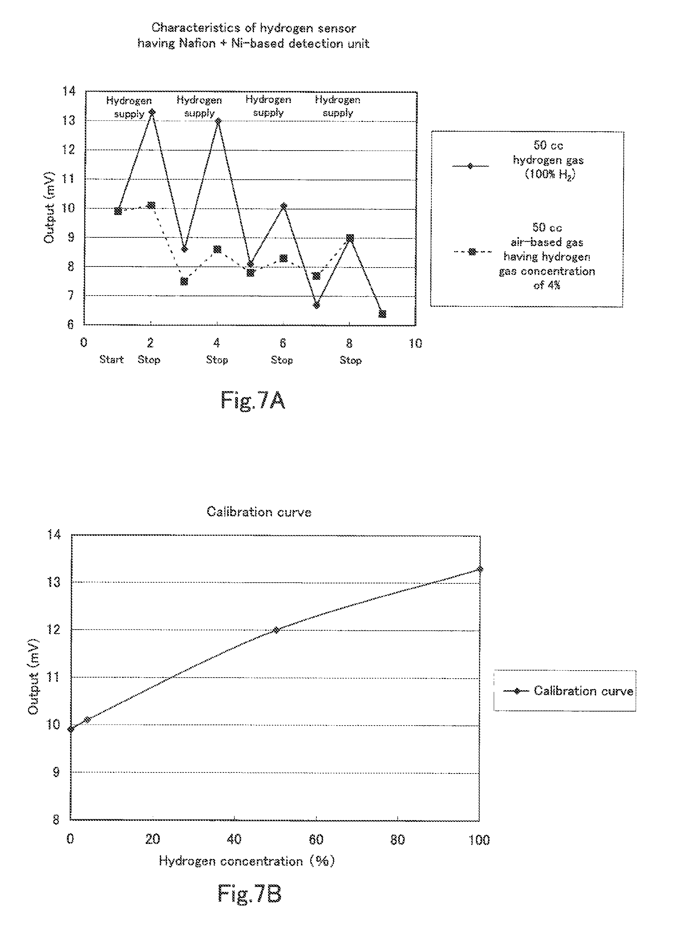

50 cc of hydrogen gas (100% H₂) and 50 cc of an air-based gas having a hydrogen gas concentration of 4% were each supplied with a syringe to the catalyst layer 3-side electrode 4a of the hydrogen gas sensor 1 at normal temperature and normal humidity (at 23°C and 55% RH), and a voltage was measured using a measuring instrument (MULTIMETER VOAC 7411). When each of the two types of gases was supplied for predetermined time and then the supply of the gas was stopped, a voltage increase of several tens of millivolts was measured. At this time, the hydrogen gas sensor of Example 1-5 exhibited the output characteristics shown in Fig. 7A and the hydrogen concentration-voltage characteristics shown in Fig. 7B.

### Example 1-6

20 mL of Poly(styrene-ran-ethylene), sulfonated solution (5 wt% 1-propanol solution) (product number: 659592) manufactured by Sigma-Aldrich Japan K.K. was heated in an oven at 100°C for condensation until the volume was reduced to 15 mL. The concentrated Poly(styrene-ran-ethylene), sulfonated solution was cast on a flat Teflon film (Teflon is a registered trademark ofDu Pont), covered with aluminum foil having a plurality of holes to evenly evaporate a solvent, and heated in an oven at 35°C until the solvent was completely evaporated. In this way, a Poly(styrene-ran-ethylene), sulfonated polymer (polystyrene-based sulfonic acid resin) film was formed as a base body 2 composed of a solid polymer electrolyte.

A piece measuring 5 cm square was cut from the thus obtained electrolyte film, and was subjected to sputtering for 30 seconds to allow platinum (Pt) to be supported as a catalyst on one of the surfaces thereof in an area measuring 4 cm square so that a catalyst layer 3 having a thickness of 20 nm was formed. In this way, a hydrogen gas sensor 1 having the same structure as shown in Fig. 3 was produced. The sputtering of platinum (Pt) was performed under the conditions where the RF power was 300 W and the flow rate of argon (Ar) gas was 20 cc/min.

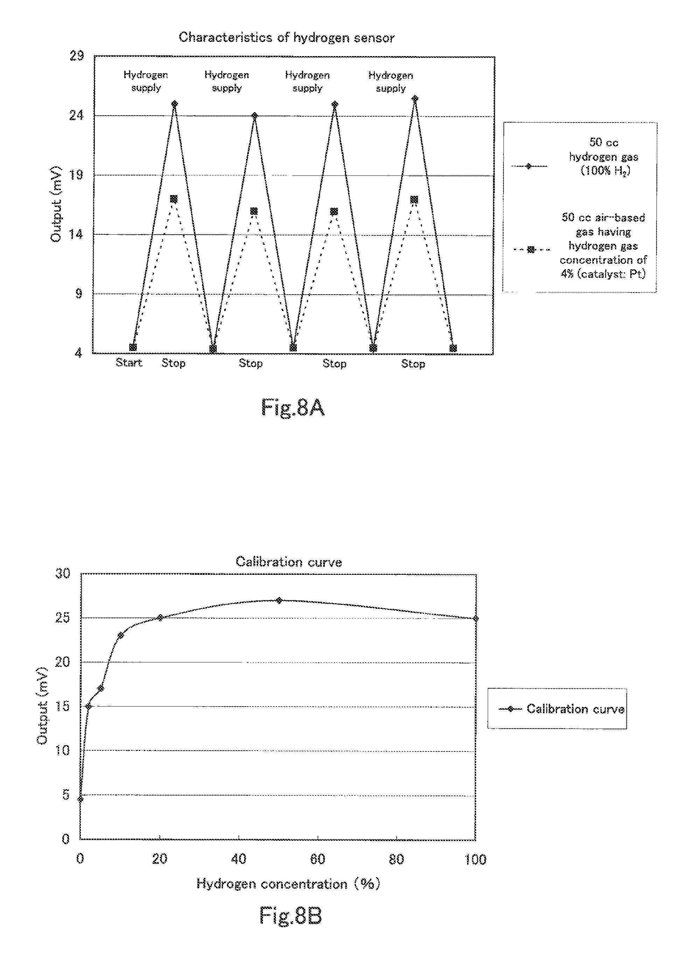

50 cc of hydrogen gas (100% H₂) and 50 cc of an air-based gas having a hydrogen gas concentration of 4% were each supplied with a syringe to the catalyst layer 3-side electrode 4a of the hydrogen gas sensor 1 at normal temperature and normal humidity (at 23°C and 55% RH), and a voltage was measured using a measuring instrument (MULTIMETER VOAC 7411). When each of the two types of gases was supplied for predetermined time and then the supply of the gas was stopped, a voltage increase of several tens of millivolts was measured. At this time, the hydrogen gas sensor of Example 1-6 exhibited the output characteristics shown in Fig. 8A and the hydrogen concentration-voltage characteristics shown in Fig. 8B.

The characteristics of the hydrogen gas sensors shown in Figs. 4 to 8 indicate that after the supply of each of the two types of gases was stopped, air flowed into the opposite electrode 4b through the openings 5a of the acrylic resin plate 5 so that hydrogen ions generated at the catalyst layer 3 were combined with oxygen contained in the air to form water.

### Example 1-7

The gas selectivity of the hydrogen gas sensor 1 obtained in Example 1-1 was evaluated. First, 40 cc of hydrogen gas (100% H₂) was brought into contact with the catalyst layer 3-side electrode 4a in the same manner as in Example 1-1, and then a voltage was measured by a measuring instrument (MULTIMETER VOAC 7411). As a result, an output voltage change of 500 mV was observed.

Then, methane gas, carbon monoxide gas, and carbon dioxide gas were each supplied to the hydrogen gas sensor 1, and a voltage was measured in the same manner as in the case of hydrogen gas. However, no output voltage change was observed in each case.

As has been described above, the hydrogen gas sensor according to the first embodiment detected an output change only when hydrogen gas was supplied. From the result, it has been found that the hydrogen gas sensor according to the first embodiment has excellent gas selectivity.

Hereinbelow, second and third embodiments of the hydrogen gas sensor according to the present invention will be described with reference to a case where a carbon-containing solid polymer electrolyte is used as a base body. It is to be noted that the description of the overlaps between the first embodiment and the second and third embodiments, such as the catalyst 3a constituting the catalyst layer 3, a method for allowing the catalyst 3a to be supported on the base body 2, the structure of the electrodes 4a and 4b, and the gas diffusion layer optionally provided, will be omitted, but a hydrogen gas sensor using a carbon-containing solid polymer electrolyte as a base body can have substantially the same structure as the above-described hydrogen gas sensor using a base body composed of only a solid polymer electrolyte.

### (Embodiment 2: First Structure Using Carbon-Containing Solid Polymer Electrolyte as Base Body)

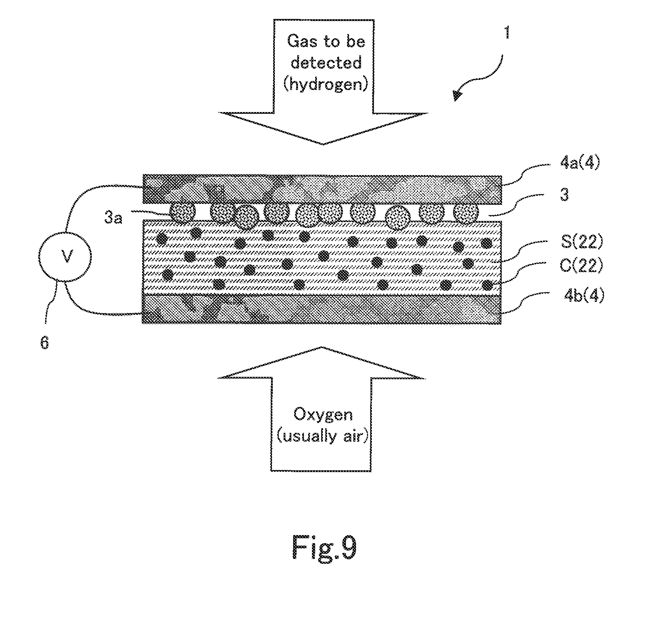

As shown in Fig. 9, the hydrogen gas sensor 1 according to a second embodiment of the present invention has a base body 22 composed of a solid polymer electrolyte S having a carbon C dispersed therein (i.e., a carbon-containing solid polymer electrolyte) and the catalyst layer 3 which performs catalytic function on contact with hydrogen gas. The catalyst layer 3 is provided on at least one of the surfaces of the base body 22. The gas-permeable electrode 4 is bonded to each of the both surfaces of the base body 22.

The solid polymer electrolyte S is not particularly limited as long as it has proton conductivity. Preferred examples of such a solid polymer electrolyte S include solid polymer electrolyte membranes composed of perfluoro-based polymers having high proton conductivity such as perfluorosulfonic acid-based polymers and perfluorocarboxylic acid-based polymers or partially sulfonated styrene-olefin copolymers such as Poly(styrene-ran-ethylene), sulfonated polymer (polystyrene-based sulfonic acid resin). More specifically, NAFION (registered trademark ofDu Pont) and ACIPLEX (registered trademark of Asahi Kasei Corporation) are preferably used.

The carbon C to be dispersed in the solid polymer electrolyte S can be selected from among carbon nanotubes (hereinafter, also referred to as "CNTs"), fullerene, graphite, nano-carbon, carbon black, nano-diamond, and nanoporous carbon. Specific examples of carbon black include furnace black, channel black, and graft carbon.

The size (particle size or length or diameter) of the carbon C (e.g., CNTs, graphite) is not particularly limited, but the length of the carbon C is preferably as long as possible. Particularly, the length of the carbon C is preferably 1 µm to 1 mm and the diameter of the carbon C is preferably 5 to 200 nm.

Further, the carbon C to be dispersed in the solid polymer electrolyte S is not limited to one type of carbon material. For example, the carbon C may be a mixture of different types of carbon materials such as a mixture of different types of CNTs and a mixture of CNTs and graphite.

More specifically, the base body 22 is composed of a carbon-containing solid polymer electrolyte obtained by mixing a solid polymer electrolyte with one or more carbon materials selected from among carbon nanotubes, fullerene, graphite, nano-carbon, carbon black, nano-diamond, and nanoporous carbon.

The base body 22 is produced through the steps of: mixing a predetermined amount of the carbon C selected from among carbon nanotubes, fullerene, graphite, nano-carbon, carbon black, nano-diamond, and nanoporous carbon with a solution of a solid polymer electrolyte obtained by dissolving a solid polymer electrolyte in an alcohol-based solvent such as a mixed water-propanol solvent; dispersing the carbon C in the solution of the solid polymer electrolyte to obtain a dispersed solution; molding the dispersed solution into a predetermined shape by, for example, casting to obtain a molded base body; and thermally drying the molded base body to remove the solvent.

The alcohol-based solvent may be changed to a polar organic solvent such as a mixed water-dimethylformamide (DMF) solvent. In either case, the dispersibility of the carbon C varies depending on the combination of a solvent used and carbon material(s) mixed with the solvent.

In the thermal drying step, the carbon C needs to be kept in a dispersion state in the base body during thermal drying. Therefore, it is important to control the heating time and temperature based on the composition of a solvent used, the weight ratio between the solid polymer electrolyte and the carbon C and the like.

The mixing ratio between the solid polymer electrolyte and the carbon C is not particularly limited as long as an electrolyte membrane in which the carbon C is dispersed can be obtained, but is preferably 10 : 1 to 10 : 10.

In a case where the above-described carbon base body is used as the base body 22, the hydrogen gas sensor has high responsivity to hydrogen gas but has low sensitivity to hydrogen gas. On the other hand, in a case where the solid polymer electrolyte described with reference to the first embodiment is used as the base body 22, the hydrogen gas sensor has high sensitivity to hydrogen gas but its fall time is long.

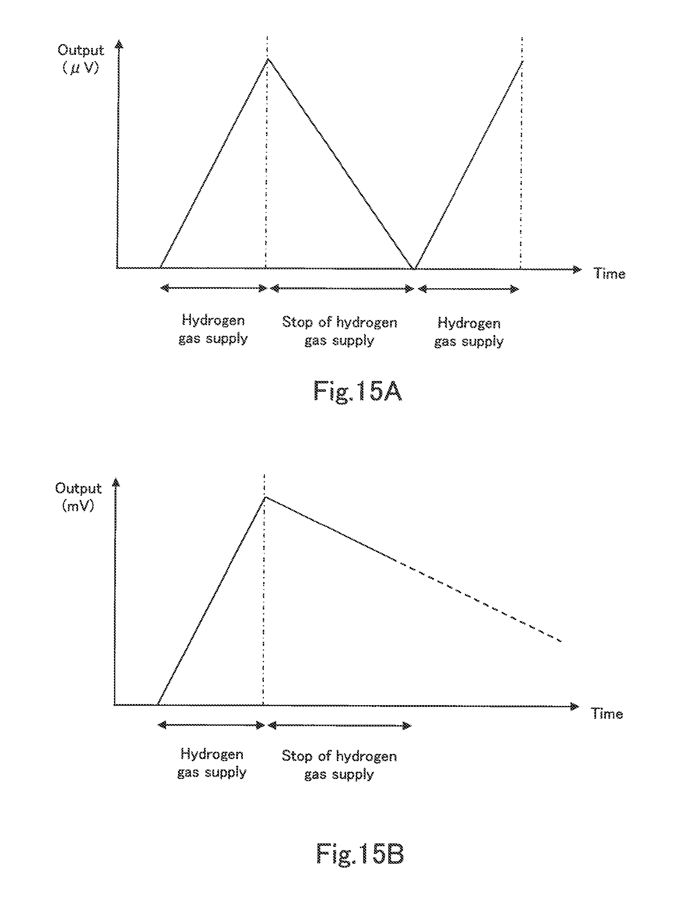

For example, as shown in Fig. 15A, when a gas having a hydrogen gas concentration of 4% is supplied at 100 cc/min to a detection unit constituted from a carbon base body measuring several centimeters per side and a platinum (Pt) catalyst layer provided on one of the surfaces of the carbon base body, electric power generation starts in several seconds; and when the supply of the hydrogen-containing gas is stopped, the production of output is also stopped in several seconds. However, in this case, the output voltage is as low as several microvolts. On the other hand, as shown in Fig. 15B, when a gas having a hydrogen gas concentration of 4% is supplied at 100 cc/min to a detection unit constituted from a solid polymer electrolyte membrane (NATION (registered trademark of Du Pont)) with the same size as the carbon base body and a platinum (Pt) catalyst layer provided on one of the surfaces of the solid polymer electrolyte membrane, electric power generation starts in several seconds and the output voltage is as high as several tens of millivolts, but the production of output is not stopped even after a lapse of long time from the stop of supply of the hydrogen-containing gas (i.e., responsivity to hydrogen gas is low).

Based on the findings, the present inventors have conceived a hydrogen gas sensor having a detection unit constituted from a base body composed of a solid polymer electrolyte having carbon dispersed therein and a catalyst layer provided on at least one of the surfaces of the base body, and have performed various experiments on the hydrogen gas sensor. As a result, it has been confirmed that such a hydrogen gas sensor is excellent in both output rise characteristics and output fall characteristics and has satisfactory sensitivity to hydrogen gas.

<Example 2: First Structure Using Carbon-Containing Solid Polymer Electrolyte as Base Body>

### Example 2-1

0.2 g of carbon was added to 10 g of Nafion 5 wt% solution, which is a solution of a solid polymer electrolyte using an alcohol-based solvent, (EC-NS-05-250 manufactured by ElectroChem, Inc.) so that the mixing ratio between Nafion and carbon was 5 : 2, and the thus obtained, mixture was stirred with a stirrer for 45 minutes to uniformly disperse the carbon. After the completion of stirring, 3 mL of the mixture was poured into a mold (5 cm × 5 cm) placed on a SUS plate, and was then heated in an oven at 120°C for 40 minutes to evaporate the solvent.

After the completion of drying, the thus obtained molded product was removed from the SUS plate. In this way, a membrane used as a base body 22 was formed. Then, the membrane was subjected to sputtering for 30 seconds to evaporate platinum (Pt) onto one of the surfaces of the membrane so that a platinum layer (catalyst layer 3) having a thickness of 20 nm was formed. The sputtering of platinum (Pt) was performed under the conditions where the RF power was 300 W and the flow rate of argon gas was 20 cc/min. It is to be noted that in Example 2-1, the back surface of the molded membrane was subjected to sputtering (here, the back surface of the molded membrane is defined as a surface brought into contact with the SUS plate), but one of the surfaces of the molded membrane to be subjected to sputtering is not limited to the back surface, and the top surface of the molded membrane may be subjected to sputtering.

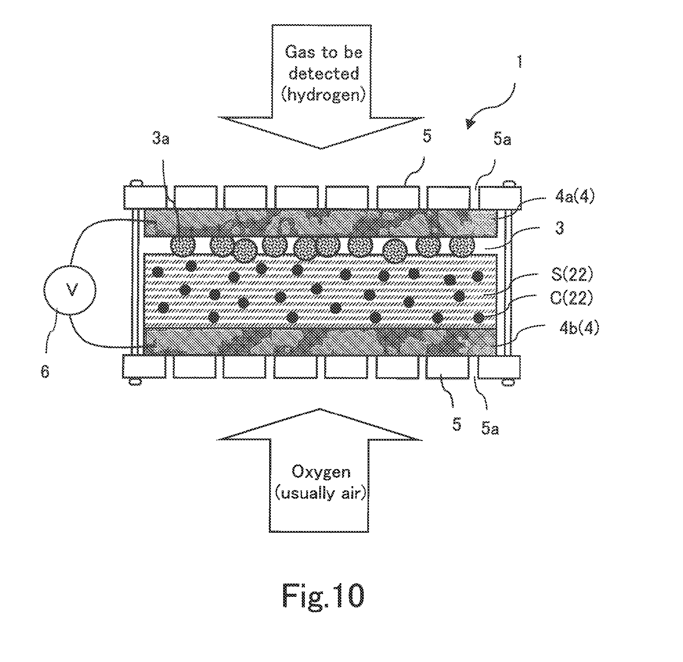

Then, as shown in Fig. 10, the base body 22 having the catalyst layer 3 provided thereon was sandwiched between a pair of electrodes 4 made of copper nickel and having a plurality of pores, and the thus obtained structure was further sandwiched between two acrylic resin plates 5 having a plurality of openings 5a. The plates 5 were fixed to each other using bolts. In this way, a hydrogen gas sensor 1 was produced.

Hydrogen gas (100% H₂), an air-based gas having a hydrogen gas concentration of 4%, and an air-based gas having a hydrogen gas concentration of 4000 ppm were each supplied at a flow rate of 100 cc/min to the catalyst layer 3-side electrode 4a of the hydrogen gas sensor 1 at normal temperature and normal humidity (at 23°C and 55% RH), and a voltage was measured using a measuring instrument (MULTIMETER VOAC 7411).

When each of the three types of gases was supplied for 3 seconds and then the supply of the gas was stopped, an output voltage was detected and generation of water was observed at the inner surface of the acrylic resin plate 5. This indicates that air flowed into the opposite electrode 4b through the openings 5a of the acrylic resin plate 5, and then hydrogen ions generated at the catalyst layer 3 were combined with oxygen contained in the air to form water.

In Example 2-1, the following carbon materials (A) to (E) (carbon nanotubes and graphite) were used as the carbon.
(A) Multi-Wall Carbon nanotubes L.MWNTs-10 manufactured by Sun Nanotech Co., Ltd.
(B) Multi-Wall Carbon nanotubes-Bundled L.B.MWNTs-10 manufactured by Sun Nanotech Co., Ltd.
(C) Ctube 100 available from Yamamoto Trading Co., Ltd.
(D) CNT and MWCNT (carbon nanotubes) manufactured by Carbon Nano-material Technology Co., Ltd.
(E) Nano Graphite manufactured by Beijing Grish Hitech Co., Ltd.

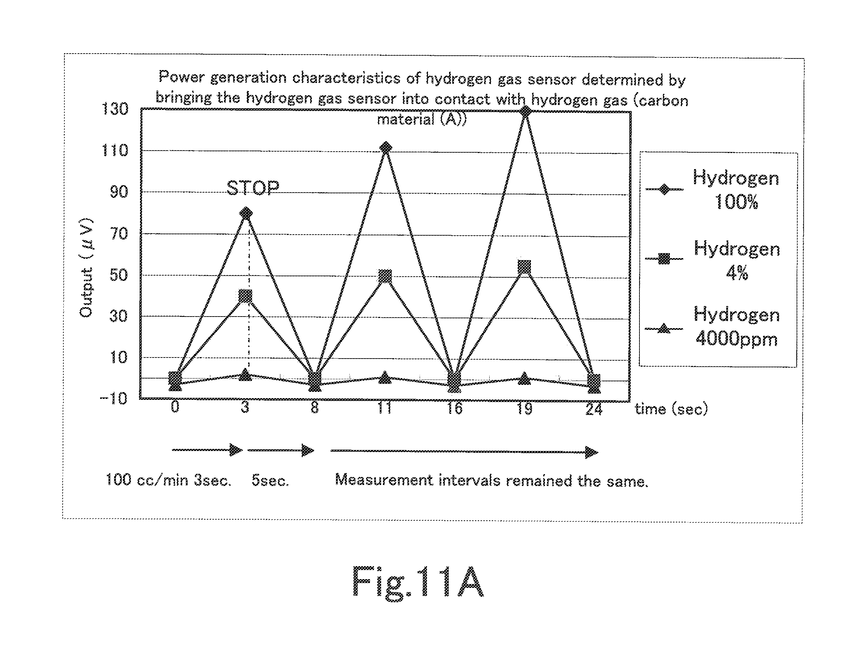

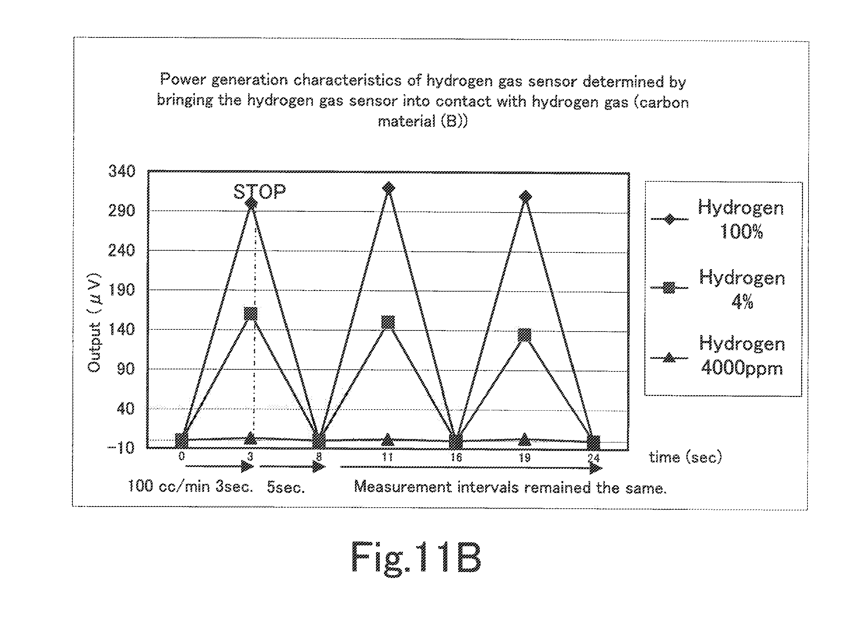

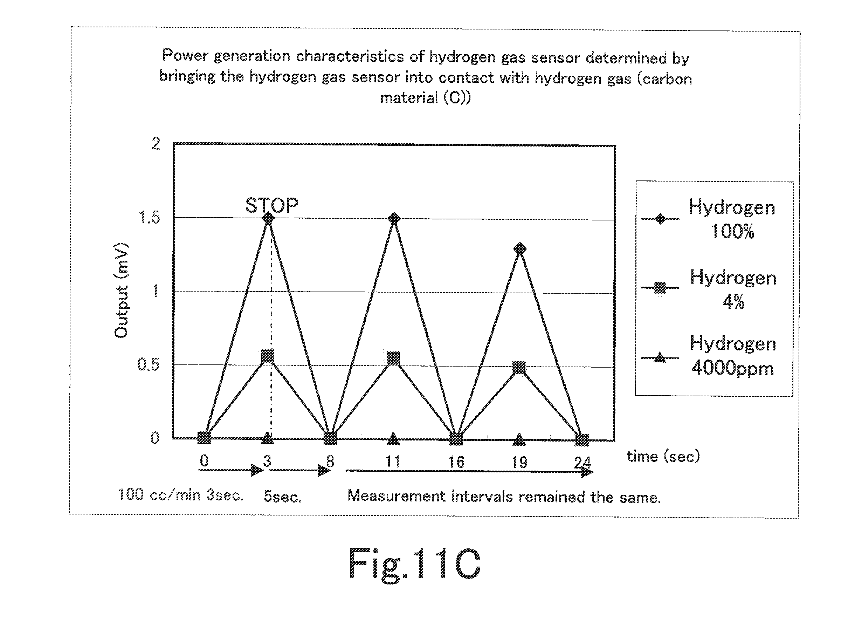

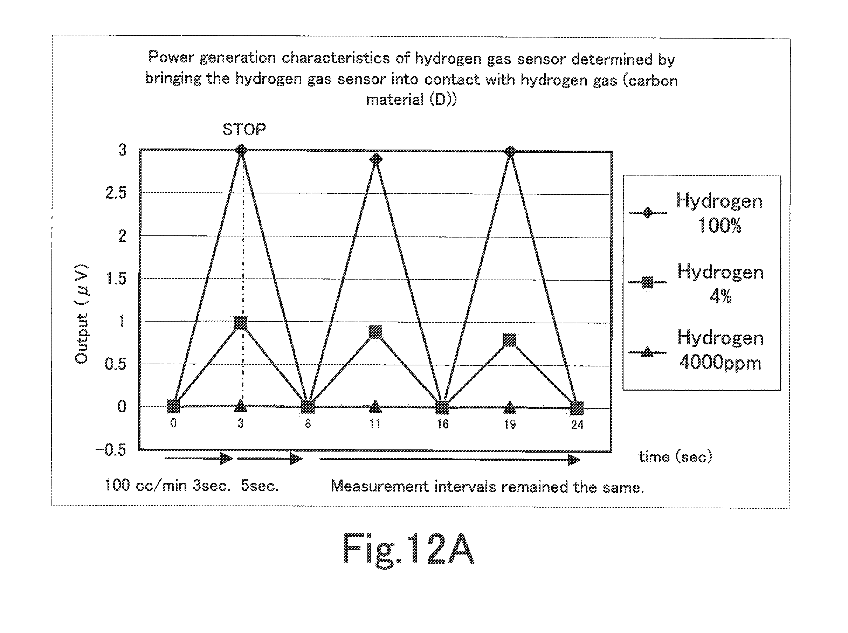

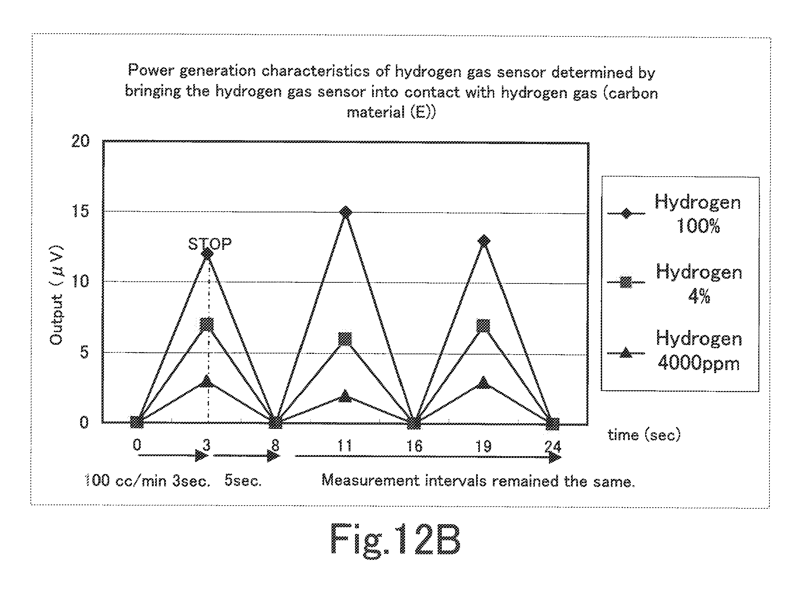

The output characteristics of the hydrogen gas sensors using the carbon materials (A) to (E) are shown in Figs. 11A to 11C and Figs. 12A and 12B, respectively. Although the hydrogen gas sensors were different in sensitivity depending on the kind of carbon material used, voltage values in the range of about several tens of microvolts to several millivolts were measured. Further, when each of the three types of gases was supplied to the hydrogen gas sensors, a voltage immediately rose; and when the supply of the gas was stopped, the voltage immediately dropped. From the result, it has been confirmed that these hydrogen gas sensors are excellent in both sensitivity and responsivity.

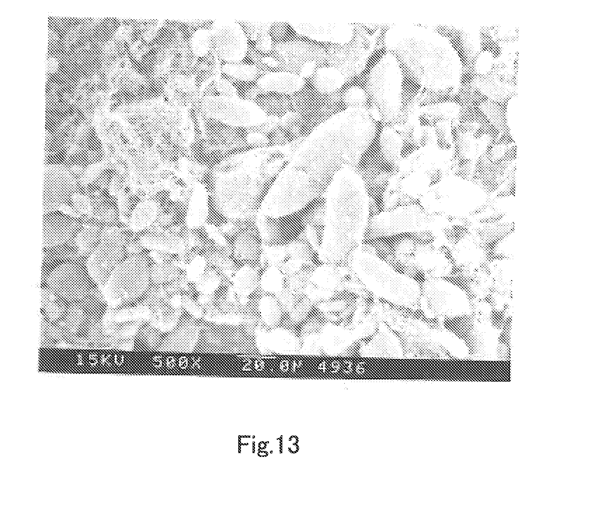

For example, in the case of the hydrogen gas sensor using the carbon nanotubes (A), as shown in Fig. 11A, when each of the three types of gases was supplied for 3 seconds, a voltage immediately rose to several tens of microvolts; and when the supply of the gas was stopped, the voltage immediately dropped in about 5 seconds. This indicates that the hydrogen gas sensor using the carbon nanotubes (A) is excellent in both sensitivity and responsivity. Fig. 13 is a scanning electron micrograph (x500) of the base body of the hydrogen gas sensor using the carbon nanotubes (A). As shown in Fig. 13, the carbon nanotubes are well mixed with and dispersed in the solid polymer electrolyte.

### Example 2-2

In the same manner as in Example 2-1, 0.2 g of the carbon material (C) of Example 2-1 was added to 10 g of a solution of a solid polymer electrolyte using an alcohol-based solvent (manufactured by Aldrich under the trade name of Poly(styrene-ran-ethylene), sulfonated, 5 wt% solution in 1-propanol), and the thus obtained mixture was stirred with a stirrer for 45 minutes to uniformly disperse the carbon material. After the completion of stirring, 3 mL of the mixture was poured into a mold (5 cm × 5 cm) placed on a SUS plate, and was then heated in an oven at 120°C for 40 minutes to evaporate the solvent.

After the completion of drying, the thus obtained molded product was removed from the SUS plate. In this way, a membrane used as a base body 22 was formed. Then, the membrane was subjected to sputtering for 30 seconds to evaporate platinum (Pt) onto one of the surfaces of the membrane so that a platinum layer (catalyst layer 3) having a thickness of 20 nm was formed. The sputtering of platinum (Pt) was performed under the conditions where the RF power was 300 W and the flow rate of argon gas was 20 cc/min. Also in Example 2-2, the back surface of the molded membrane was subjected to sputtering.

Similarly, the base body 22 having the catalyst layer 3 provided thereon was sandwiched between a pair of electrodes 4 made of copper nickel and having a plurality of pores, and the thus obtained structure was further sandwiched between two acrylic resin plates 5 having a plurality of openings 5a. The plates 5 were fixed to each other using bolts. In this way, a hydrogen gas sensor 1 was produced.

Hydrogen gas (100% H₂), an air-based gas having a hydrogen gas concentration of 4%, and an air-based gas having a hydrogen gas concentration of 4000 ppm were each supplied at a flow rate of 100 cc/min to the catalyst layer 3-side electrode 4a of the hydrogen gas sensor 1 at normal temperature and normal humidity (at 23°C and 55% RH), and a voltage was measured using a measuring instrument (MULTIMETER VOAC 7411).

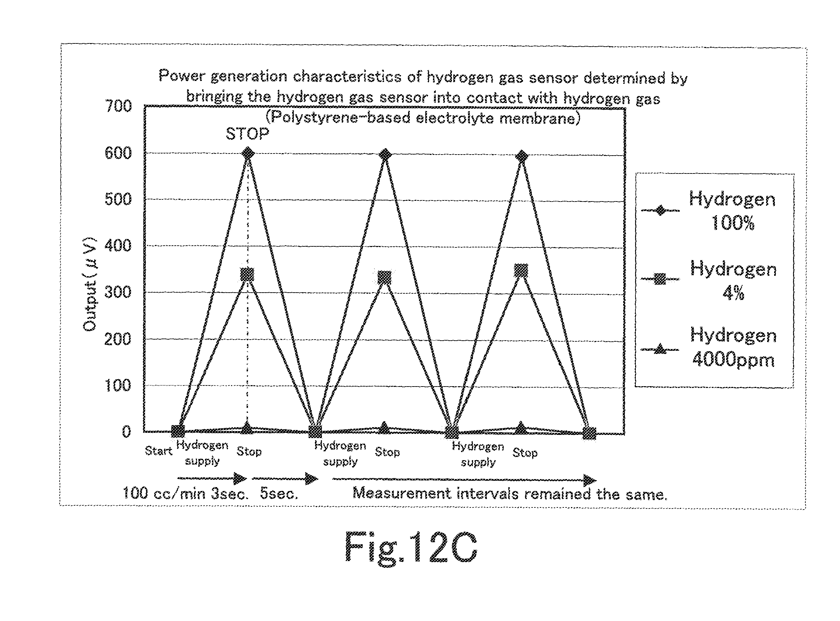

The output characteristics of the hydrogen gas sensor of Example 2-2 are shown in Fig. 12C. Although the solid polymer electrolyte of the hydrogen gas sensor of Example 2-2 was different from that of the hydrogen gas sensor of Example 2-1, a voltage increase of about several hundreds of microvolts was measured. Further, when each of the three types of gases was supplied to the hydrogen gas sensor of Example 2-2, a voltage immediately rose; and when the supply of the gas was stopped, the voltage immediately dropped. From the result, it has been confirmed that the hydrogen gas sensor of Example 2-2 is excellent in both sensitivity and responsivity.

### Example 2-3

A verification test was performed on some base bodies whose power generation characteristic had been previously determined in order to examine the influence of water on the hydrogen gas sensor according to the present invention.

A Nafion membrane (sample 1) having the carbon material (CNTs) (A) used in Example 2-1 dispersed therein and containing Nafion and the CNTs in a ratio of 5 : 2, a baked membrane (sample 2) formed using the carbon material (C) used in Example 2-1, and a Nafion membrane (sample 3) were prepared as base bodies. Then, each of the three types of base bodies was subjected to sputtering to evaporate platinum (Pt) onto one of the surfaces thereof under the same conditions as in the above examples to obtain a detection unit. The thus obtained detection unit was picked up with tweezers, and was then immersed in water for 5 seconds. Then, a hydrogen gas sensor 1 shown in Fig. 10 was produced using the detection unit, and a verification test was performed on the hydrogen gas sensor 1 to determine a change in the behavior of the hydrogen gas sensor 1 during the detection of hydrogen before and after immersion in water.

The baked membrane prepared as a sample 2 was formed in the following manner. 10 wt% of the Ctube 100 and 30 wt% of a polypyrrole resin (CDP-310M manufactured by Japan Carlit Co., Ltd.) were mixed with N-methylpyrrolidone as an organic solvent (hereinafter, simply referred to as "NMP") to obtain a mixture, and the mixture was shaken using a paint shaker for 60 minutes to prepare a carbon nanotube dispersed solution. The carbon nanotube dispersed solution was cast on a SUS plate having a partition measuring 5 cm square previously provided thereon to obtain a sheet-shaped molded product having a thickness of about 250 µm. The thus obtained molded product was dried and then baked in a baking machine at about 600°C for 1 hour under a nitrogen gas atmosphere to thermally decompose and vaporize the binder resin and NMP. In this way, a baked membrane was obtained.

The Nafion membrane prepared as a sample 3 was formed in the following manner. A piece measuring 5 cm square was cut from a Nafion membrane having a thickness of 100 µm (NRE-212 manufactured by Du Pont), and then the piece of Nafion membrane was sandwiched between two sheets of filter paper and pressed from above with an iron heated to 130°C to remove water. The piece of Nafion membrane was repeatedly heated using the iron until no water could penetrate through the filter paper.

The power generation characteristics of the hydrogen gas sensors before and after immersion in water determined by bringing the hydrogen gas sensors into contact with hydrogen gas (100% H₂) are shown in Figs. 14A to 14C. As can be seen from Fig. 14B, the power generation characteristics of the hydrogen gas sensor using the CNT baked membrane (sample 2) as a base body were seriously affected by immersion in water. In addition, it has been also found that the shape retentivity of the CNT baked membrane used as a base body is deteriorated by immersion in water. Further, as shown in Fig. 14C, the output fall characteristics of the hydrogen gas sensor using the Nafion membrane (sample 3) as a base body were affected by immersion in water. From the result, it is supposed that the hydrogen gas sensor using the Nafion membrane (sample 3) cannot properly function as a sensor depending on its purpose of use.

On the other hand, the sensitivity of the hydrogen gas sensor using the Nafion membrane having CNTs dispersed therein (sample 1) as a base body was improved by immersion in water, and the output rise characteristics and output fall characteristics of the hydrogen gas sensor using the Nafion membrane (sample 1) as a base body were not changed before and after immersion in water.

### Example 2-4

A hydrogen gas sensor 1 was produced in the same manner as in Example 2-1 except that the carbon was changed to 0.2 g of fullerene (nanom mix ST-F manufactured by Frontier Carbon Corporation) and fullerene was added to 10 g of Nafion 5 wt% solution, which is a solution of a solid polymer electrolyte using an alcohol-based solvent, (EC-NS-05-250 manufactured by ElectroChem, Inc.) so that the mixing ratio between Nafion and carbon was 5 : 1 2.

When 40 cc of an air-based gas having a hydrogen gas concentration of 4% was brought into contact with the catalyst layer 3-side electrode 4a of the hydrogen gas sensor 1 at normal temperature and normal humidity (at 23°C and 55% RH), an output voltage measured by a measuring instrument (MULTIMETER VOAC 7411) was changed from -256 mV to 381 mV.

### Example 2-5

Nafion 5 wt% solution, which is a solution of a solid polymer electrolyte using an alcohol-based solvent, (FC-NS-05-250 manufactured by ElectroChem, Inc.) was heated in an evaporator at 60°C to evaporate the IPA solvent. After the vaporization of IPA was completed, the operation of the evaporator was stopped with water remaining in the evaporator. Then, a DMF solvent was added thereto to extract Nafion with DMF. In this way, a solution of a solid polymer electrolyte using a mixed water-DMF solvent was obtained.

Then, a hydrogen gas sensor 1 was produced in the same manner as in Example 2-1 except that the carbon was changed to 0.2 g of fullerene (nanom mix ST-F manufactured by Frontier Carbon Corporation) and fullerene was added to 10 g of Nafion 5 wt% solution, which is a solution of a solid polymer electrolyte using the above-described mixed water-DMF solvent so that the mixing ratio between Nafion and carbon was 5 : 2.

When 40 cc of an air-based gas having a hydrogen gas concentration of 4% was brought into contact with the catalyst layer 3-side electrode 4a of the hydrogen gas sensor 1 at normal temperature and normal humidity (at 23°C and 55% RH), an output voltage measured by a measuring instrument (MULTIMETER VOAC 7411) was changed from -300 mV to 18 mV.

### Example 2-6

A hydrogen gas sensor 1 was produced in the same manner as in Example 2-1 except that the carbon was changed to 0.2 g of diamond powder UFD-WP1 (manufactured by Beijing Grish Hitech Co., Ltd.) and diamond powder was added to 10 g of Nafion 5 wt% solution, which is a solution of a solid polymer electrolyte using an alcohol-based solvent, (EC-NS-05-250 manufactured by ElectroChem, Inc.) so that the mixing ratio between Nafion and carbon was 5 : 2.

When 40 cc of an air-based gas having a hydrogen gas concentration of 4% was brought into contact with the catalyst layer 3-side electrode 4a of the hydrogen gas sensor 1 at normal temperature and normal humidity (at 23°C and 55% RH), an output voltage measured by a measuring instrument (MULTIMETER VOAC 7411) was changed from -10 mV to 18 mV.

### Example 2-7

The gas selectivity of the hydrogen gas sensor 1 obtained in Example 2-5 was evaluated. First, 40 cc of hydrogen gas (100% H₂) was brought into contact with the catalyst layer 3-side electrode 4a in the same manner as in Example 2-5, and then a voltage was measured by a measuring instrument (MULTIMETER VOAC 7411). As a result, an output voltage change of 200 mV was observed.

Then, methane gas, carbon monoxide gas, and carbon dioxide gas were each supplied to the hydrogen gas sensor 1, and a voltage was measured in the same manner as in the case of hydrogen gas. However, no output voltage change was observed in each case.

As has been described above, the hydrogen gas sensor according to the second embodiment detected an output change only when hydrogen gas was supplied. From the result, it has been found that the hydrogen gas sensor according to the second embodiment has excellent gas selectivity.

### (Embodiment 3: Second Structure Using Carbon-Containing Solid Polymer Electrolyte as Base Body)

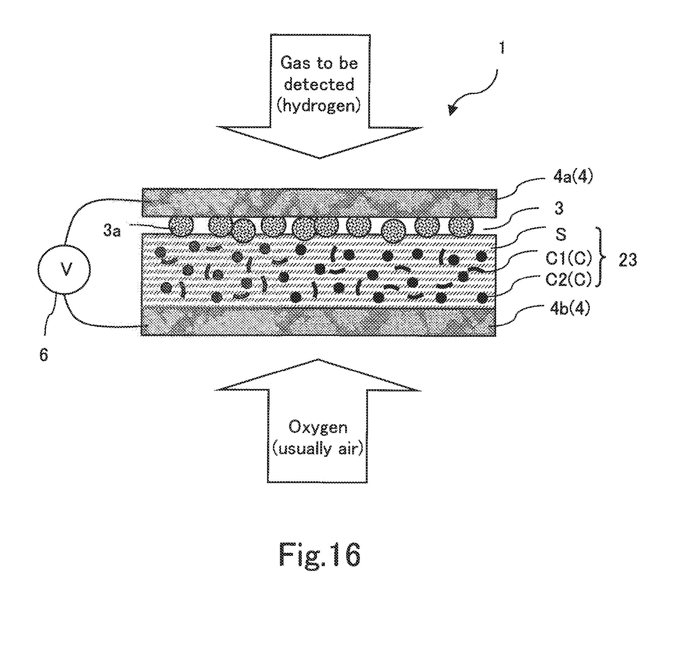

As shown in Fig. 16, the hydrogen gas sensor 1 according to a third embodiment of the present invention has a base body 23 composed of a solid polymer electrolyte S in which a carbon C containing at least two types of carbon materials is dispersed (i.e., a carbon-containing solid polymer electrolyte) and the catalyst layer 3 which performs catalytic function on contact with hydrogen gas. The catalyst layer 3 is provided on at least one of the surfaces of the base body 23. The gas-permeable electrode 4 is bonded to each of the both surfaces of the base body 23.

The solid polymer electrolyte S to be used for forming the base body 23 is not particularly limited as long as it has proton conductivity. Preferred examples of such a solid polymer electrolyte S include solid polymer electrolyte membranes composed of perfluoro-based polymers having high proton conductivity such as perfluorosulfonic acid-based polymers and perfluorocarboxylic acid-based polymers or hydrocarbon-based sulfonic acid resins such as partially sulfonated styrene-olefin copolymers (e.g., Poly(styrene-ran-ethylene), sulfonated polymer (polystyrene-based sulfonic acid resin)). More specifically, NAFION (registered trademark of Du Pont) and ACIPLEX (registered trademark of Asahi Kasei Corporation) are preferably used.

The carbon C to be dispersed in the solid polymer electrolyte S is a mixture of at least one carbon C1 selected from the group consisting of carbon nanotubes, graphite, nano-carbon, and carbon black and at least one carbon C2 selected from the group consisting of fullerene, nano-diamond, and nanoporous carbon. Particularly, a mixture of carbon nanotubes and fullerene or nano-diamond or a mixture of graphite and fullerene or nano-diamond is preferred.

The size of the carbon C1 such as carbon nanotubes or graphite is not particularly limited, but the length of the carbon C1 is preferably as long as possible. Particularly, the length of the carbon C1 is preferably 1 µm to 1 mm, and the diameter of the carbon C1 is preferably 5 to 200 nm.

The spherical carbon C2 selected from among fullerene, nano-diamond, and nanoporous carbon preferably has an average particle size of 8 to 5000 nm.

More specifically, the base body 23 is composed of a carbon-containing solid polymer electrolyte obtained by mixing a solid polymer electrolyte with at least one carbon material selected from the group consisting of carbon black, graphite, nano-carbon, and carbon nanotubes and at least one carbon material selected from the group consisting of fullerene, nano-diamond, and nanoporous carbon.

The percentage by weight X of the carbon C2 contained in the carbon mixture C is previously set according to the target range of a hydrogen gas concentration to be detected.

For example, in a case where the detection unit is produced using a base body containing a carbon mixture C obtained by mixing a carbon C2 selected from the group consisting of fullerene, nano-diamond, and nanoporous carbon so that the percentage by weight X of the carbon C2 with respect to the carbon mixture C becomes 50 or more but less than 100 (50 ≤ X < 100), preferably 65 or more but 85 or less (65 ≤ X ≤ 85), the hydrogen gas sensor produces a relatively stable output in the absence of hydrogen gas, and has high sensitivity and therefore can detect a hydrogen gas concentration as low as only 0.04%.

Such a hydrogen gas sensor can be used as, for example, a room sensor for fuel-cell cars or a medical hydrogen gas sensor (which is used to detect hydrogen in the oral cavity).

On the other hand, in a case where the detection unit is produced using a base body containing a carbon mixture C obtained by mixing a carbon C2 selected from the group consisting of fullerene, nano-diamond, and nanoporous carbon so that the percentage by weight X of the carbon C2 with respect to the carbon mixture C becomes more than 0 but less than 50 (0 < X < 50), preferably 20 or more but 40 or less (20 ≤ X ≤ 40), output stability of the hydrogen gas sensor in the absence of hydrogen gas can be ensured, and the hydrogen gas sensor can properly detect a hydrogen gas concentration as high as 4% or more.

Such a hydrogen gas sensor can be used as, for example, a stationary hydrogen gas sensor for hydrogen stations or a gas leakage detection unit to be provided near hydrogen storage tanks in fuel-cell cars or near fuel cells.

That is, by adjusting the mixing ratio between carbon material(s) selected from the group consisting of carbon nanotubes, graphite, nano-carbon, and carbon black and carbon material(s) selected from the group consisting of fullerene, nano-diamond, and nanoporous carbon contained in the base body according to the target range of a hydrogen gas concentration to be detected, the hydrogen gas sensor can have appropriate sensitivity to accurately detect a hydrogen gas concentration while the output stability of the hydrogen gas sensor in the absence of hydrogen gas can be ensured.

The base body 23 is produced through the steps of mixing a solution of a solid polymer electrolyte obtained by dissolving the solid polymer electrolyte S in an alcohol-based solvent such as isopropanol with a predetermined amount of the carbon mixture C obtained by mixing at least one carbon C1 selected from the group consisting of carbon nanotubes, graphite, nano-carbon, and carbon black and at least one carbon C2 selected from the group consisting of fullerene, nano-diamond, and nanoporous carbon in a predetermined weight ratio; dispersing the carbon mixture C in the solution of the solid polymer electrolyte to obtain a dispersed solution; molding the dispersed solution into a predetermined shape by, for example, casting to obtain a molded base body; and thermally drying the molded base body to remove the solvent.

The alcohol-based solvent may be changed to a polar organic solvent such as a mixed water-dimethylformamide (DMF) solvent.

The dispersion of the carbon C1 and the carbon C2 in the solution of the solid polymer electrolyte can be achieved by a well-known method. Examples of such a well-known method include, but are not limited to, a method using a stirrer, a paint shaker, or a homogenizer and an ultrasonic dispersion method.

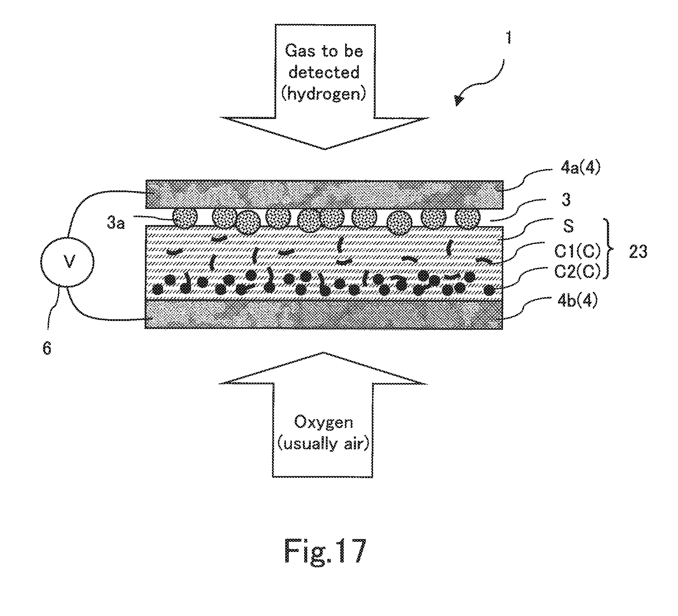

In the thermal drying step, the heating time and temperature are preferably controlled based on the composition of a solvent used, the weight ratio between the solid polymer electrolyte S and the carbon mixture C, and the like so that a predetermined dispersion state of the carbon mixture C can be achieved. Particularly, as shown in Fig. 17, the heating time and temperature are preferably controlled so that it is possible to obtain a thermally-dried base body in which the carbon C1 having a specific gravity equal to or smaller than that of the solution of the solid polymer electrolyte is uniformly dispersed and the carbon C2 having a specific gravity larger than that of the solution of the solid polymer electrolyte is dispersed in an area near one of the surfaces (usually, near the lower surface) thereof. By doing so, it is possible to obtain a base body having preferred characteristics.

That is, the base body 23 having a thin film shape preferably has a bilayer structure achieved by uniformly dispersing the carbon C1 having a specific gravity equal to or smaller than that of the solution of the solid polymer electrolyte in the base body 23 throughout its thickness and by dispersing the carbon C2 having a specific gravity larger than that of the solution of the solid polymer electrolyte in the lower-side portion of the base body 23 in the thickness direction.

The mixing ratio between the solid polymer electrolyte S and the carbon mixture C is not particularly limited as long as an electrolyte membrane, in which the carbon mixture C is dispersed in such a state as described above, can be obtained, but is preferably 10 : 1 to 10 : 10.

In a case where the base body 23 has such a bilayer structure as described above, the catalyst layer 3 is preferably provided on the upper layer-side of the base body 23, but may be provided on the lower layer-side of the base body 23.

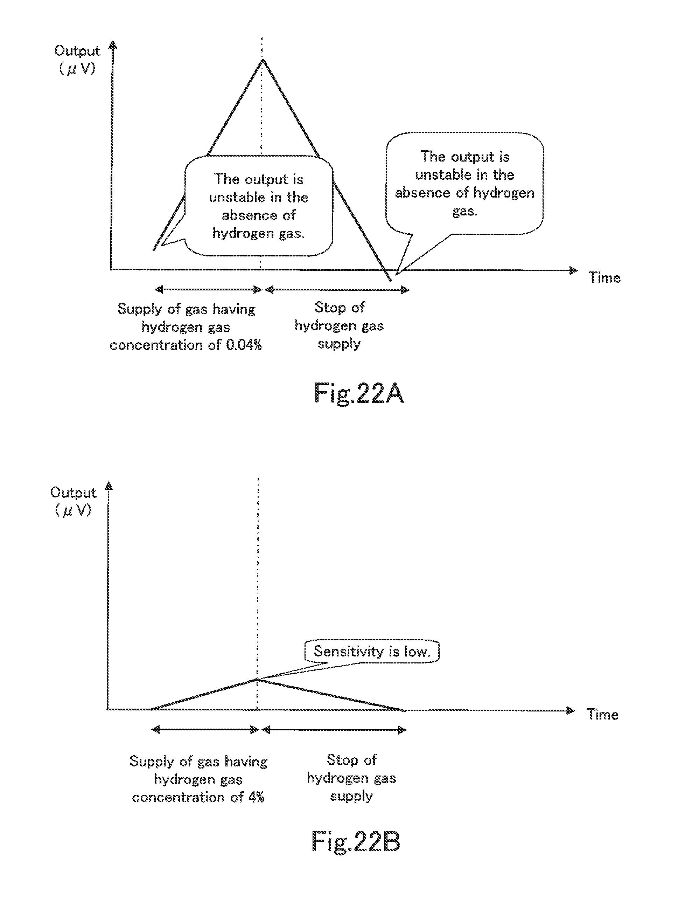

As a result of an extensive study, the present inventors have found that when the detection unit is produced using a base body containing carbon selected from the group consisting of fullerene, nano-diamond, and nanoporous carbon, as shown in Fig. 22A, the hydrogen gas sensor has sensitivity to a gas having a very low hydrogen gas concentration, but is saturated when a gas having a high hydrogen gas concentration is supplied and therefore cannot accurately detect a hydrogen gas concentration. Further, the present inventors have also found that such a hydrogen gas sensor tends to fluctuate in output (i.e., tends to show low output stability) in the early stage in the absence of hydrogen gas, which may be due to high impedance.

Further, the present inventors have also found that when the detection unit is produced using a base body containing carbon selected from the group consisting of carbon nanotubes, graphite, nano-carbon, and carbon black, as shown in Fig. 22B, the output stability of the hydrogen gas sensor in the absence of hydrogen gas can be ensured, but the hydrogen gas sensor tends to have sensitivity only to a gas having a relatively high hydrogen gas concentration.

Based on these findings, the present inventors have newly found that when the detection unit is produced using a base body obtained by mixing a solid polymer electrolyte with at least one carbon material selected from the group consisting of carbon nanotubes, graphite, nano-carbon, and carbon black and at least one carbon material selected from the group consisting of fullerene, nano-diamond, and nanoporous carbon, the sensitivity of the hydrogen gas sensor to hydrogen gas can be appropriately controlled by adjusting the ratio (i.e., percentage by weight X) of the carbon material (s) selected from the group consisting of fullerene, nano-diamond, and nanoporous carbon with respect to the total carbon materials mixed, while the output stability of the hydrogen gas sensor in the absence of hydrogen gas can be ensured.

The base body having a bilayer structure can be formed also by mixing a solution of a solid polymer electrolyte with two types of carbon materials showing different dispersibility in a solvent contained in the solution of the solid polymer electrolyte.

For example, the base body 23 can be produced through the steps of: mixing a solution of a solid polymer electrolyte with two types of carbon materials different in dispersibility in a solvent used in a predetermined weight ratio; dispersing the carbon materials in the solution of the solid polymer electrolyte to obtain a dispersed solution; molding the dispersed solution into a predetermined shape by, for example, casting to obtain a molded base body; and thermally drying the molded base body to remove the solvent.

Carbon materials can be divided into two groups according to the kind of solvent used. One of the two groups includes carbon materials showing high dispersibility and the other includes carbon materials showing low dispersibility.

An example of a combination of two types of carbon materials showing different dispersibility in a solvent used includes the above-described combination of at least one carbon C1 selected from the group consisting of carbon nanotubes, graphite, nano-carbon, and carbon black and at least one carbon C2 selected from the group consisting of fullerene, nano-diamond, and nanoporous carbon. It has been already found that the use of such a combination leads to a relatively good result.

As in the case of the above-described base body, the use of such a base body obtained by mixing a solution of a solid polymer electrolyte with two types of carbon materials showing different dispersibility in a solvent contained in the solution of the solid polymer electrolyte makes it possible to appropriately control the sensitivity of the hydrogen gas sensor to hydrogen gas. Also in this case, a solvent contained in the solution of the solid polymer electrolyte may be either an alcohol-based solvent or a polar organic solvent such as dimethylformamide (DMF).

### <Example 3: Second Structure Using Carbon-Containing Solid Polymer Electrolyte as Base Body>

### Example 3-1

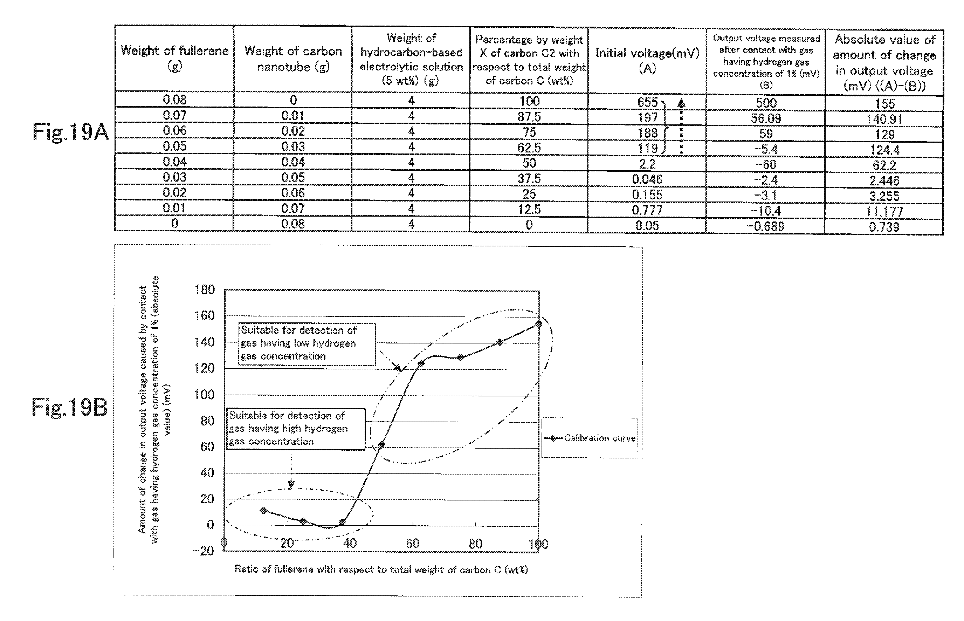

A solution of a solid polymer electrolyte using an alcohol-based solvent (manufactured by Aldrich under the trade name of Poly(styrene-ran-ethylene), sulfonated, 5 wt% solution in 1-propanol), C60/C70-mixed fullerene (manufactured by Frontier Carbon Corporation under the trade name of nanom mix ST-F) used as a carbon C2, and carbon nanotubes (available from Yamamoto Trading Co., Ltd. under the trade name of C100) used as a carbon C1 were prepared. As shown in Fig. 19A, various carbon mixtures C different in the ratio (i.e., percentage by weight X, where 0 ≤ X ≤ 100) of the carbon C2 with respect to the total carbon materials mixed were prepared by mixing the carbon C1 and the carbon C2 so that the total mass of the carbon C and the carbon C2 was 0.08 g. Each of the carbon mixtures C was mixed with 4 g of the solution of the solid polymer electrolyte and stirred with a stirrer for 1 hour.

After the completion of stirring, each of the solutions of solid polymer electrolyte containing the carbon mixture C dispersed therein was poured into a SUS mold (3 cm × 3 cm) and dried at 90°C for 2 hours to evaporate the solvent so that a base body 23 was formed.

After the completion of drying, the base body 23 was removed from the mold, and platinum (Pt) was sputtered onto the casting surface of the base body 23 to form a catalyst layer 3. In this way, a detection unit was produced. The sputtering of platinum (Pt) was performed under the conditions where the RF power was 300 W and the flow rate of argon gas was 20 cc/min. After the completion of sputtering, the detection unit was cut to have a size of 22 mm × 22 mm. In this way, various detection units uniform in size were produced.

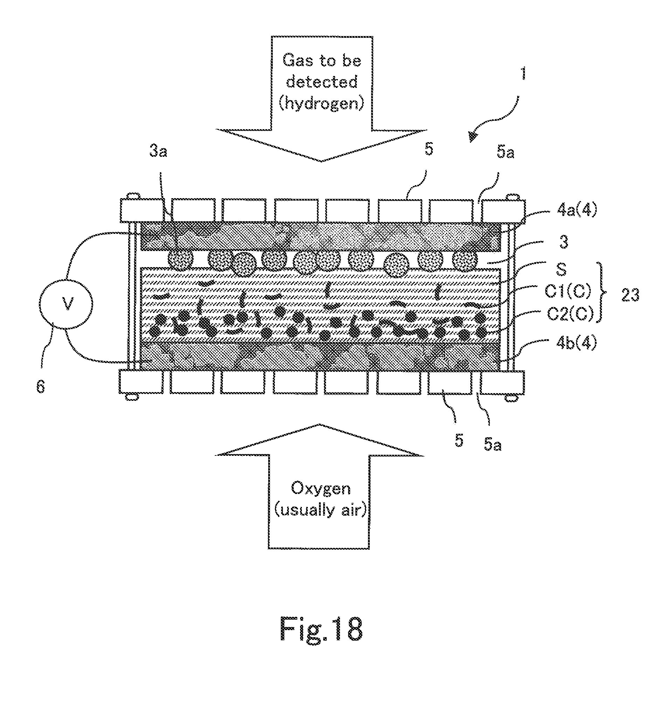

As shown in Fig. 18, the base body 23 having the catalyst layer 3 provided thereon was sandwiched between a pair of electrodes 4 made of copper nickel and having a plurality of pores, and the thus obtained structure was further sandwiched between two acrylic resin plates 5 having a plurality of openings 5a. The plates 5 were fixed to each other using bolts. In this way, a hydrogen gas sensor 1 was produced.

Various prototypes of the hydrogen gas sensor 1 were produced using the different detection units prepared above. Each of the prototypes of the hydrogen gas sensor 1 was brought into contact with a gas having a hydrogen gas concentration of 1% to determine the output change before and after the contact with hydrogen gas.

The output characteristics of the hydrogen gas sensor 1, that is, the characteristics between output and the percentage by weight X of the fullerene C2 with respect to the total weight of the carbon mixture C of the hydrogen gas sensor 1 are shown in Fig. 19B.

### Example 3-2

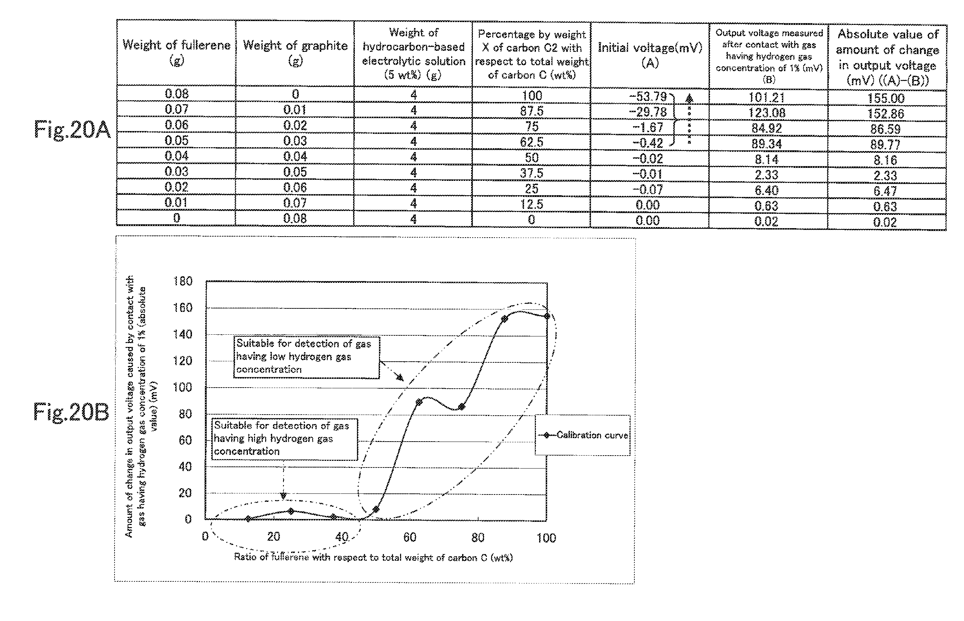

A solution of a solid polymer electrolyte (manufactured by Aldrich under the trade name of Poly(styrene-ran-ethylene), sulfonated, 5 wt% solution in 1-propanol), C60/C70-mixed fullerene (manufactured by Frontier Carbon Corporation under the trade name of nanom mix ST-F) used as a carbon C2, and graphite (Lot. G60326, manufactured by Beijing Grish Hitech Co., Ltd.) used as a carbon C1 were prepared. As shown in Fig. 20A, various carbon mixtures C different in the ratio (i.e., percentage by weight X, where 0 ≤ X ≤ 100) of the carbon C2 with respect to the total carbon materials mixed were prepared by mixing the carbon C 1 and the carbon C2 so that the total mass of the carbon C1 and the carbon C2 was 0.08 g. Each of the carbon mixtures C was mixed with 4 g of the solution of the solid polymer electrolyte and stirred with a stirrer for 1 hour.

After the completion of stirring, each of the solutions of solid polymer electrolyte containing the carbon mixture C dispersed therein was poured into a SUS mold (3 cm × 3 cm) and dried at 90°C for 2 hours to evaporate the solvent so that a base body 23 was formed.

After the completion of drying, the base body 23 was removed from the mold, and platinum (Pt) was sputtered onto the casting surface of the base body 23 to form a catalyst layer 3. In this way, a detection unit was produced. The sputtering of platinum (Pt) was performed under the conditions where the RF power was 300 W and the flow rate of argon gas was 20 cc/min. After the completion of sputtering, the detection unit was cut to have a size of 22 mm × 22 mm. In this way, various detection units uniform in size were produced.

In the same manner as in Example 3-1, as shown in Fig. 18, the base body 23 having the catalyst layer 3 provided thereon was sandwiched between a pair of electrodes 4 made of copper nickel and having a plurality of pores, and the thus obtained structure was further sandwiched between two acrylic resin plates 5 having a plurality of openings 5a. The plates 5 were fixed to each other using bolts. In this way, a hydrogen gas sensor 1 was produced.

Various prototypes of the hydrogen gas sensor 1 were produced using the different detection units prepared above. Each of the prototypes of the hydrogen gas sensor 1 was brought into contact with a gas having a hydrogen gas concentration of 1% to determine the output change before and after the contact with hydrogen gas.

The output characteristics of the hydrogen gas sensor 1, that is, the characteristics between output and the percentage by weight X of the fullerene C2 with respect to the total weight of the carbon mixture C of the hydrogen gas sensor 1 are shown in Fig. 20B.

As shown in Fig. 19B and Fig. 20B, in both cases of Examples 3-1 and 3-2, the sensitivity of the hydrogen gas sensor 1 to hydrogen gas varied depending on the percentage by weight X of the fullerene C2 with respect to the total weight of the carbon mixture C.

The dashed arrows in Fig. 19A and Fig. 20A indicate that a higher positive or negative initial potential is detected as the percentage by weight X of the fullerene increases, and therefore it takes longer time before the output of the hydrogen gas sensor 1 becomes stable.

From the results shown in Figs. 19A, 19B, 20A, and 20B, it has been confirmed that when the detection unit is produced using a base body, in which a carbon mixture C obtained by mixing the fullerene C2 so that the percentage by weight X of the fullerene C2 with respect to the total weight of the carbon mixture C becomes 50 or more but less than 100 (i.e., 50 ≤ X < 100), preferably 65 or more but 85 or less (i.e., 65 ≤ X ≤ 85) is dispersed, the hydrogen gas sensor 1 has sensitivity to a gas having a very low hydrogen gas concentration and produces a relatively stable output in the absence of hydrogen gas.

In addition, it has been also confirmed that when the detection unit is produced using a base body, in which a carbon mixture C obtained by mixing the fullerene C2 so that the percentage by weight X of the fullerene C2 with respect to the total weight of the carbon mixture C becomes more than 0 but less than 50 (i.e., 0 < X < 50), preferably 20 or more but 40 or less (i.e., 20 ≤ X ≤ 40) is dispersed, the hydrogen gas sensor 1 can properly detect a gas having a high hydrogen gas concentration while the output stability of the hydrogen gas sensor 1 in the absence of hydrogen gas can be ensured.

### Example 3-3

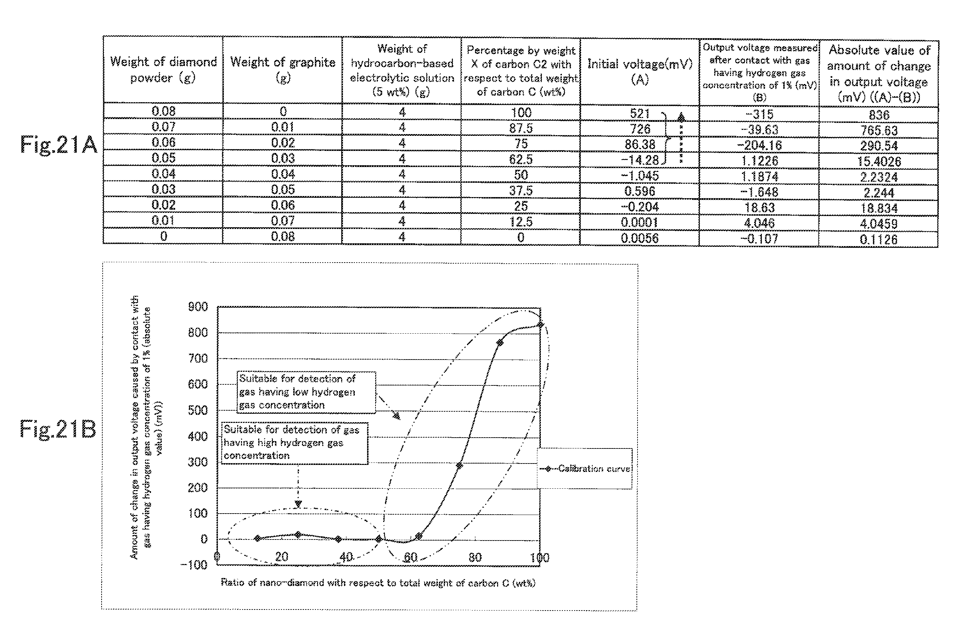

A solution of a solid polymer electrolyte (manufactured by Aldrich under the trade name of Poly(styrene-ran-ethylene), sulfonated, 5 wt% solution in 1-propanol), diamond powder (UFD-WP1, manufactured by Beijing Grish Hitech Co., Ltd.) used as a carbon C2, and graphite (Lot. G60326, manufactured by Beijing Grish Hitech Co., Ltd.) used as a carbon C1 were prepared. As shown in Fig. 21A, various carbon mixtures C different in the ratio (i.e., percentage by weight X, where 0 ≤ X ≤ 100) of the carbon C2 with respect to the total carbon materials mixed were prepared by mixing the carbon C1 and the carbon C2 so that the total mass of the carbon C1 and the carbon C2 was 0.08 g. Each of the carbon mixtures C was mixed with 4 g of the solution of the solid polymer electrolyte and stirred with a stirrer for 1 hour.

After the completion of stirring, each of the solutions of solid polymer electrolyte containing the carbon mixture C dispersed therein was poured into a SUS mold (3 cm × 3 cm) and dried at 90°C for 2 hours to evaporate the solvent so that a base body 23 was formed.

After the completion of drying, the base body 23 was removed from the mold, and platinum (Pt) was sputtered onto the casting surface of the base body 23 to form a catalyst layer 3. In this way, a detection unit was produced. The sputtering of platinum (Pt) was performed under the conditions where the RF power was 300 W and the flow rate of argon gas was 20 cc/min. After the completion of sputtering, the detection unit was cut to have a size of 22 mm × 22 mm. In this way, various detection units uniform in size were produced.

As shown in Fig. 18, the base body 23 having the catalyst layer 3 provided thereon was sandwiched between a pair of electrodes 4 made of copper nickel and having a plurality of pores, and the thus obtained structure was further sandwiched between two acrylic resin plates 5 having a plurality of openings 5a. The plates 5 were fixed to each other using bolts. In this way, a hydrogen gas sensor 1 was produced.

Various prototypes of the hydrogen gas sensor 1 were produced, using the different detection units prepared above. Each of the prototypes of the hydrogen gas sensor 1 was brought into contact with a gas having a hydrogen gas concentration of 1%, to determine the output change before and after the contact with hydrogen gas.

The output characteristics of the hydrogen gas sensor 1, that is, the characteristics between output and the mixing ratio between nano-diamond and graphite are shown in Fig. 21B.

As shown in Fig. 21B, the sensitivity of the hydrogen gas sensor 1 to hydrogen gas varied depending on the percentage by weight X of the nano-diamond C2 with respect to the total weight of the carbon mixture C.

The dashed arrow in Fig. 21A indicates that a higher positive or negative initial potential is detected as the percentage by weight X of the narw-diamond increases, and therefore it takes longer time before the output of the hydrogen gas sensor 1 becomes stable.

From the results shown in Figs. 21A and 21B, it has been confirmed that when the detection unit is produced using a base body, in which a carbon mixture C obtained by mixing the nano-diamond C2 so that the percentage by weight X of the nano-diamond C2 with respect to the total weight of the carbon mixture C becomes 50 or more but less than 100 (i.e., 50 ≤ X < 100), preferably 65 or more but 85 or less (i.e., 65 ≤ X ≤ 85) is dispersed, the hydrogen gas sensor 1 has sensitivity to a gas having a very low hydrogen gas concentration and produces a relatively stable output in the absence of hydrogen gas.

In addition, it has been also confirmed that when the detection unit is produced using a base body, in which a carbon mixture C obtained by mixing the nano-diamond C2 so that the percentage by weight X of the nano-diamond C2 with respect to the total weight of the carbon mixture C becomes more than 0 but less than 50 (i.e., 0 < X < 50), preferably 20 or more but 40 or less (i.e., 20 ≤ X ≤ 40) is dispersed, the hydrogen gas sensor 1 can properly detect a gas having a high hydrogen gas concentration while the output stability of the hydrogen gas sensor 1 in the absence of hydrogen gas can be ensured.

### Example 3-4

0.1 g of carbon nanotubes (Ctube 100 available from Yamamoto Trading Co., Ltd.) and 0.1 g of fullerene (nanom mix ST-F manufactured by Frontier Carbon Corporation) were mixed with 0.5 g of a solution of a solid polymer electrolyte using a mixed water-DMF solvent obtained in the same manner as in Example 2-5, and then the thus obtained mixture was stirred. Then, a hydrogen gas sensor 1 was produced in the same manner as described above.

When 40 cc of hydrogen gas (100% H₂) was brought into contact with the catalyst layer 3-side electrode 4a of the hydrogen gas sensor 1 at normal temperature and normal humidity (at 23°C and 55% RH), an output voltage measured by a measuring instrument (MULTIMETER VOAC 7411) was changed from 1 µmV to 33 mV.

### Example 3-5

The gas selectivity of the hydrogen gas sensor 1 obtained in Example 3-4 was evaluated. First, 40 cc of hydrogen gas (100% H₂) was brought into contact with the catalyst layer 3-side, electrode 4a in the same manner as in Example 3-4, and then a voltage was measured by a measuring instrument (MULTIMETER VOAC 7411). As a result, an output voltage change of 33 mV was observed.

Then, methane gas, carbon monoxide gas, and carbon dioxide gas were each supplied to the hydrogen gas sensor 1, and a voltage was measured in the same manner as in the case of hydrogen gas. However, no output voltage change was observed in each case.

Was has been described above, the hydrogen gas sensor according to the third embodiment detected an output change only when hydrogen gas was supplied. From the result, it has been found that the hydrogen gas sensor according to the third embodiment has excellent gas selectivity.

### < Embodiment 4: Structure Using Molybdenum Carbide Mo₂C as Catalyst>

Hereinbelow, the hydrogen gas sensor according to a fourth embodiment of the present invention will be described with reference to a case where the catalyst layer is made of molybdenum carbide (Mo₂C). Further, concrete examples of how the hydrogen gas sensor is used will be also described.

A combination of the hydrogen gas sensor and a control unit can be used as a hydrogen gas sensor system for fuel-cell systems. Such a hydrogen gas sensor system is intended to be provided adjacent to the fuel gas distribution channel of a fuel cell to give warning to the FC (Fuel Cell) control unit of a fuel cell system based on the output of the hydrogen gas sensor for detecting a hydrogen gas concentration when the concentration of hydrogen gas leaked from the distribution channel in ambient atmosphere reaches a certain level (e.g., 4000 ppm (1/10 of 4% that is the explosion limit of hydrogen)) or higher.

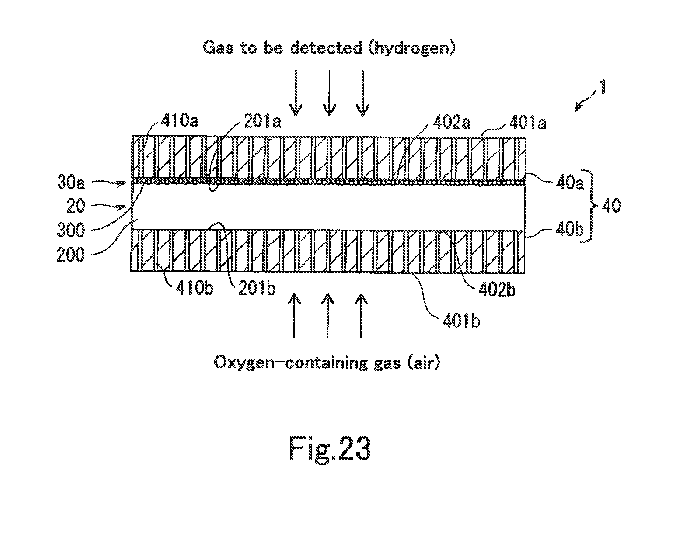

As shown in Fig. 23, the hydrogen gas sensor 1 is constituted from a sheet-shaped detection unit 20 and an electrode pair 40. The electrode pair 40 is provided on both surfaces of the detection unit 20 so that the detection unit 20 is sandwiched between the electrode pair 40. The detection unit 20 has a base body formed from a solid polymer electrolyte membrane 200 and a catalyst layer 30a provided on a main surface 201a which is one of the two surfaces of the base body.

The solid polymer electrolyte membrane 200 has the function of allowing protons separated from hydrogen by the catalyst layer 30a to pass through it from the main surface 201a to the other main surface 201b. The solid polymer electrolyte membrane 200 is composed of a solid polymer electrolyte having proton conductivity, and has a sheet shape with a thickness of about 100 µm, a length of 5 cm, and a width of 5 cm.

As has been described above, the solid polymer electrolyte is preferably a perfluoro-based polymer having high proton conductivity such as a perfluorosulfonic acid-based polymer or a perfluorocarboxylic acid-based polymer or a hydrocarbon-based polymer.

The catalyst layer 30a has the function of catalyzing a hydrogen decomposition reaction when coming into contact with hydrogen gas contained in ambient atmosphere. It is to be noted that in this embodiment, the catalyst layer 30a is made of 100% molybdenum carbide (Mo₂C) instead of expensive platinum (Pt). More specifically, the catalyst layer 30a is formed by a thin film forming method such as sputtering, an EB method, a CVD method, or a PVD method using Mo₂C catalyst particles 300 so as to have a thickness of about 4 to 60 nm.

The electrode pair 40 is provided to take out output resulting from a hydrogen decomposition reaction occurring at the catalyst layer 30a and the amount of change in output to the outside. The electrode pair 40 includes a hydrogen-side electrode 40a provided in contact with the catalyst layer 30a of the detection unit 20 and an oxygen-side electrode 40b provided in contact with the solid polymer electrolyte membrane 200. Examples of the material of these electrodes include metal materials having good electric conductivity such as copper-nickel alloy thin films.

The electrodes 40a and 40b have a plurality of fine perforations 410a and 410b obtained by perforating the main surfaces thereof in the thickness direction to supply hydrogen gas and an oxygen-containing gas (usually air) to the detection unit 20. The hydrogen-side electrode 40a is brought into contact with an analyte gas (hydrogen gas), and the oxygen-side electrode 40b is brought into contact with an oxygen-containing gas.

The electrodes 40a and 40b are not limited to those having the fine perforations 410a and 410b respectively as long as they have good electric conductivity and the ability to allow gas to pass through them to supply gas to the inside of the hydrogen gas sensor. For example, the electrodes 40a and. 40b may be formed from a porous metal sintered body or carbon-containing fibers such as carbon paper or carbon cloth. Among them, carbon paper and carbon cloth have proton conductivity and particularly excellent gas permeability, and are therefore preferred as electrode materials.

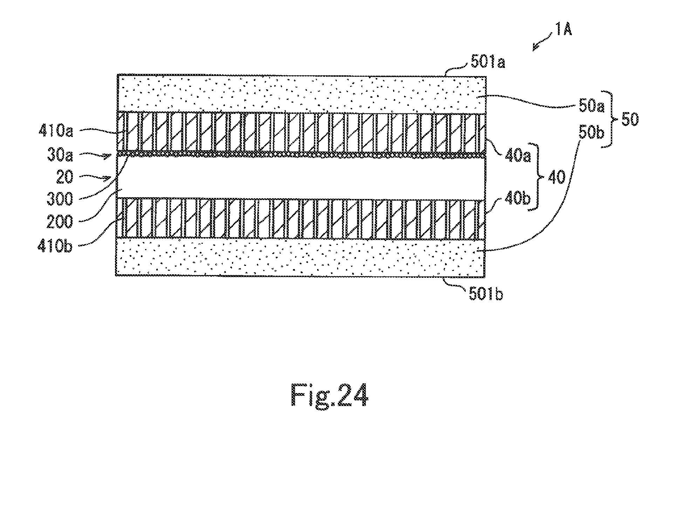

The hydrogen gas sensor 1 may further have a gas diffusion layer. As shown in Fig. 24, a hydrogen gas sensor 1A has a gas diffusion layer 50 provided on the electrodes 40a and 40b. The gas diffusion layer 50 is formed from carbon paper or carbon cloth or the like. By providing gas diffusion layers 50a and 50b, it is possible to diffuse hydrogen gas (air) to efficiently supply the hydrogen gas (air) from a main surface 501a (501b) of the gas diffusion layer 50a (50b) to the hydrogen-side electrode 40a (oxygen-side electrode 40b). Therefore, it can be expected that the use of such a gas diffusion layer will lead to good operation of the sensor 1.

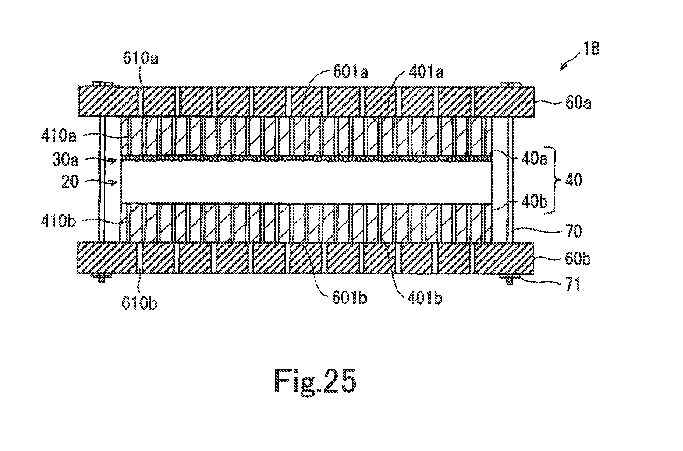

Alternatively, the hydrogen gas sensor 1 shown in Fig. 23 may further have perforated plates to promote gas distribution. As shown in Fig. 25, a hydrogen gas sensor 1B has perforated plates having a plurality of fine perforations 610a and 610b respectively (in this case, acrylic resin plates 60a and 60b are used). The hydrogen gas sensor 1B has a stack structure achieved by stacking the perforated plates 60a and 60b on the electrodes 40a and 40b of the hydrogen gas sensor 1 shown in Fig. 23 respectively so that main surfaces 601a and 601b of the perforated plates 60a and 60b come into contact with the main surfaces 401a and 401b of the electrodes 40a and 40b respectively. The perforated plates 60a and 60b are fixed to each other using bolts 70 and nuts 71.

It is to be noted that the hydrogen gas sensor 1B may be produced by sandwiching the hydrogen gas sensor 1A shown in Fig. 24 instead of the hydrogen gas sensor 1 shown in Fig. 23 between the perforated plates to achieve a stack structure. This makes it easy to handle the hydrogen gas sensor 1B. In addition, such a stack structure is advantageous in that a plurality of hydrogen gas sensors can be stacked together.

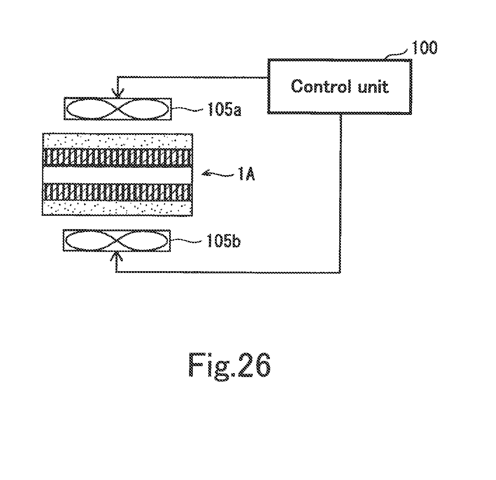

Further, as shown in Fig. 26, well-known cooling fans 105a and 105b may be provided near the electrodes 40a and 40b of the hydrogen gas sensor 1 or the gas diffusion layers 50a and 50b of the hydrogen gas sensor 1A respectively so that the cooling fan 105a is opposed to the electrode 40a or the gas diffusion layer 50a and the cooling fan 105b is opposed to the electrode 40b or the gas diffusion layer 50b.

The driving of the fans 105a and 105b is controlled by a control unit 100 separately provided. The fans 105a and 105b constitute a gas sending system for continuously or intermittently sending an analyte gas and an oxygen-containing gas (usually air) to the hydrogen-side electrode 40a and the oxygen-side electrode 40b respectively. The use of such a gas sending system can be expected to lead to higher-accuracy detection of hydrogen gas by the hydrogen gas sensor 1 or 1A.

When sending an analyte gas and air to the hydrogen gas sensor 1, the gas sending system can be operated in mode A where an analyte gas and air are sent to the hydrogen gas sensor 1 at the same time or mode B where an analyte gas is first sent to the hydrogen gas sensor 1 and then air is sent to the hydrogen gas sensor 1 after a temporary stop. Further, the gas sending system can be operated in mode C where air is sent to the hydrogen gas sensor 1 at a certain pressure to remove contamination or dust attached to the gas diffusion layers or the like by air pressure and to eliminate unnecessary gas retained in the hydrogen gas sensor 1. It is to be noted that the mode C can be combined with the mode A or B.

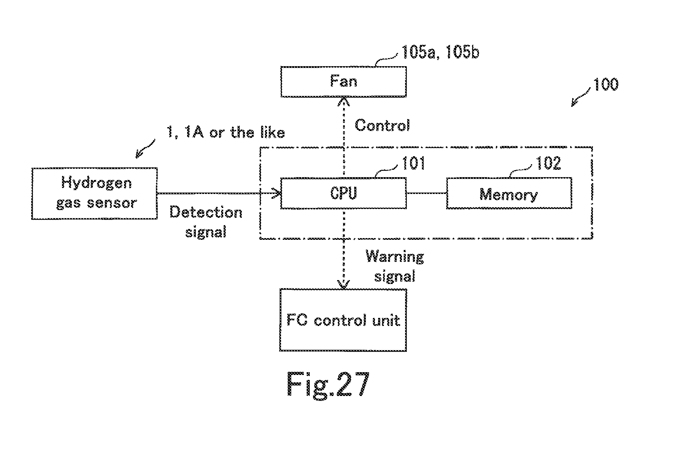

As shown in Fig. 27, the control unit 100 is connected as an external circuit to the electrode pair 40 to monitor a voltage value or a current value as a detection signal from the hydrogen gas sensor 1. The control unit 100 has a CPU 101 and a memory 102 incorporated therein.

The CPU 101 monitors a detection signal from the hydrogen gas sensor 1 based on a hydrogen gas sensor control program stored in the memory 102. When the detection signal exceeds a certain level, the CPU 101 sends a warning signal to the FC control unit to stop the operation of the fuel cell system. Further, the CPU 101 controls the driving of the fans 105a and 105b based on a fan control program stored in the memory 102 so that the fans 105a and 105b can be operated in the mode A, B, or C.

As described above, the memory 102 stores the program for controlling the hydrogen gas sensor 1 and the program for controlling the fans 105a and 105b. Further, the memory 102 stores information about the reference value of a hydrogen gas concentration corresponding to a current value or a voltage value as a detection signal from the hydrogen gas sensor 1. The CPU 101 reads out these control programs and information from the memory 102 when necessary.

When hydrogen gas comes into contact with the catalyst layer 30a of the hydrogen gas sensor 1, a hydrogen decomposition reaction occurs due to the catalytic action of the catalyst particles 300 composed of molybdenum carbide (Mo₂C) so that hydrogen molecules (H₂) are split into protons and electrons (H₂ → 2H⁺ + 2e⁻).

The electrons generated by the hydrogen decomposition reaction flow through the external circuit connected to the hydrogen-side electrode 40a, and are then inputted into the CPU 101 of the control unit 100 as a detection signal to calculate a hydrogen gas concentration.

On the other hand, water generated near the oxygen-side electrode is partially lost by evaporation near the boundary between the oxygen-side electrode 40b and the solid polymer electrolyte membrane 200, and the remaining water is used to keep the solid polymer electrolyte membrane 200 wet. This makes it possible for the solid polymer electrolyte membrane 200 to keep good proton conductivity.

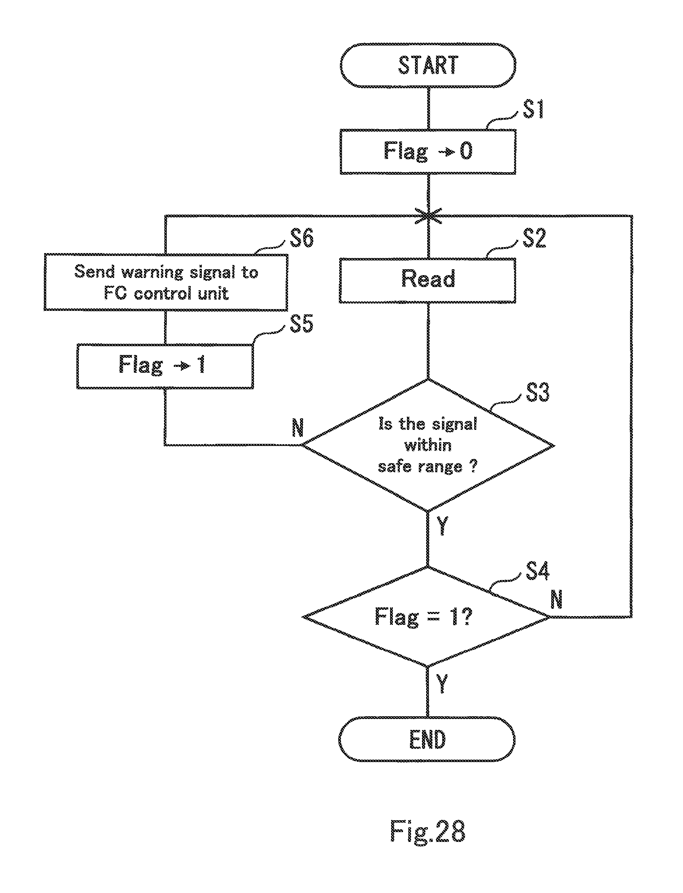

Hereinbelow, the operation of the hydrogen gas sensor system will be described based on a flow chart shown in Fig. 28.

When the fuel cell system and the hydrogen gas sensor system are driven, the CPU 101 initializes control data or the like and at this time a flag is set to 0 (S1). Then, the CPU 101 continuously reads a detection signal from the hydrogen gas sensor 1 (S2) and compares the value of the detection signal to the reference value (a current or voltage value of the sensor 1 corresponding to a hydrogen gas concentration less than 4000 ppm) previously stored in the memory 102 to determine whether or not a hydrogen gas concentration detected by the hydrogen gas sensor 1 is within a safe range (S3).

When the CPU 101 determines that the hydrogen gas concentration is within a safe range, the CPU 101 further determines whether or not the flag is 1 (S4). When the flag is reset to 0, the CPU 101 returns to step S2. That is, the CPU 101 repeats a control flow including steps S2 to S4 as long as the detection signal is within a safe range.

On the other hand, when the CPU 101 determines that the hydrogen gas concentration exceeds a safe range (S3), the flag is set to 1 (S5) and a warning signal is sent to the FC control unit of the fuel cell system (S6). At this time, the CPU 101 repeats a control flow including steps S2 to S6 until the CUP 101 again determines that a hydrogen gas concentration corresponding to a detection signal from the hydrogen gas sensor 1 is within a safe range (S3) as a result, of some kind of action taken on the fuel cell system by a user.

When the CPU 101 again determines that a hydrogen gas concentration detected by the hydrogen gas sensor 1 is within a safe range (S3) and then the CPU determines that the flag is 1 (S4), the CPU 101 terminates the control flow to stop the hydrogen gas sensor system.

When the situation where a warning signal is sent to the FC control unit occurs, the fuel cell system is stopped by a user or the FC control unit to make inspections or secure safety.

The control flow shown in Fig. 28 is based on the assumption that when the control unit 100 sends a warning signal for hydrogen gas leakage to the FC control unit, the FC control unit performs stop control to stop the fuel cell system. However, if the fuel cell system is not stopped, steps S1, S4, and S5 are omitted, and a control flow including steps S2, S3, and S6 is repeated to constantly monitor a hydrogen gas concentration when the hydrogen gas sensor system is running.

Hereinbelow, advantages gained by using molybdenum carbide (Mo₂C) as a catalyst constituting the catalyst layer 30a will be described.

Molybdenum carbide (Mo₂C) is much cheaper than platinum (Pt) conventionally and widely used as a catalyst. More specifically, the market price of a molybdenum carbide sputtering target is about 1/20 of that of a platinum (Pt) sputtering target.

Therefore, by forming the catalyst layer using molybdenum carbide (Mo₂C) as an alternative to platinum (Pt), it is possible to significantly reduce the production cost of the hydrogen gas sensor as compared to a case where the catalyst layer is formed using platinum (Pt). This is advantageous in that the productivity of the hydrogen gas sensor is drastically enhanced than ever before.

Further, the use of molybdenum carbide (Mo₂C) as a catalyst makes it possible to readily contemplate an increase in the amount of a catalyst to be used in the hydrogen gas sensor.

Generally, the gas detection performance of a hydrogen gas sensor is proportional to the area of contact between a catalyst and hydrogen gas, and therefore can be improved by increasing the amount of a catalyst. That is, an increase in the amount of a catalyst used leads to an increase in the area of contact between a catalyst and hydrogen gas, thereby making it possible to detect hydrogen gas with high accuracy.

Even if the catalytic performance of molybdenum carbide (Mo₂C) is regarded as being slightly lower than that of platinum (Pt), since molybdenum carbide (Mo₂C) is cheap, the gap in the catalytic performance can be easily covered by increasing the amount of molybdenum carbide (Mo₂C) used as a catalyst.

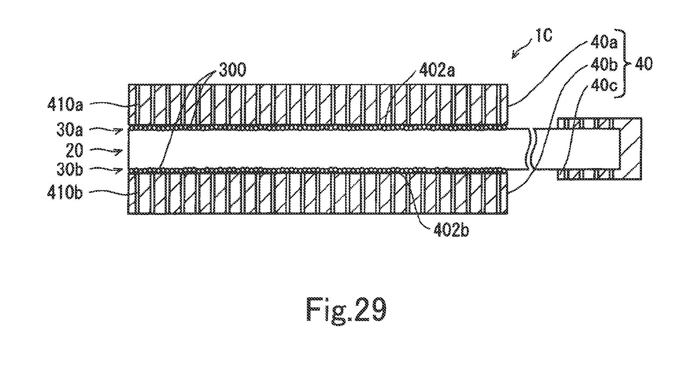

For example, as shown in Fig. 29, a hydrogen gas sensor 1C has catalyst layers 30a and 30b obtained by allowing molybdenum carbide (Mo₂C) to be supported on both surfaces 402a and 402b of the solid polymer electrolyte membrane 200. In this case, the electrodes 40a and 40b are both anodes, and a cathode 40c is separately provided at the end of the solid polymer electrolyte membrane. This makes it possible to provide a large hydrogen detection region constituted from the catalyst layers provided on the both surfaces of the solid polymer electrolyte membrane 200.

As has been described above, since molybdenum carbide (Mo₂C) is cheap, the amount of molybdenum carbide (Mo₂C) used as a catalyst is not limited in terms of price. Therefore, molybdenum carbide (Mo₂C) can be expected to offer catalytic performance comparable to that of platinum (Pt).

Further, the use of molybdenum carbide (Mo₂C) as a catalyst is advantageous also in that the hydrogen gas detection characteristics of the hydrogen gas sensor are improved as compared to a case where platinum (Pt) is used as a catalyst.

Platinum (Pt) exhibits its excellent catalytic action on contact with hydrogen gas. In this case, however, once a hydrogen decomposition reaction occurs, the reaction continues to occur for some time. Therefore, once a hydrogen gas sensor using platinum (Pt) as a catalyst detects hydrogen gas, an unnecessary hydrogen decomposition reaction continues to occur due to remaining hydrogen present around the platinum catalyst. This makes it impossible to speedily reduce the output level of the hydrogen gas sensor even after a hydrogen gas concentration in the hydrogen gas sensor is reduced.

The present inventors have found by experiments that when a hydrogen sensor obtained by evaporating platinum (Pt) onto a Nafion membrane as a solid polymer electrolyte membrane by sputtering for 30 seconds is brought into contact with hydrogen gas (100% H₂), the fall time (i.e., the time required to return the hydrogen sensor to its normal output state to allow the hydrogen sensor to be reset and restarted) of the hydrogen sensor is about 10 minutes, thus resulting in the loss of time.

On the other hand, the present inventors have found that a hydrogen gas sensor using molybdenum carbide (Mo₂C) as a catalyst does not excessively react with hydrogen and therefore returns to its normal output state in several seconds after contact with hydrogen gas. Such a hydrogen gas sensor having excellent responsivity to hydrogen gas is practically very useful, and can be used as a high-concentration hydrogen sensor.

Further, it is known that the use of platinum (Pt) as a catalyst causes a problem that the surface of a catalyst is covered with carbon monoxide attached thereto so that the catalytic activity of the catalyst is lowered. This problem is so-called "CO poisoning". Such CO poisoning deprives a catalyst of its catalytic activity and deteriorates the hydrogen detection performance of a hydrogen gas sensor.

However, in a case where molybdenum carbide (Mo₂C) is used as a catalyst, the influence of reduction in catalytic activity caused by CO poisoning is smaller as compared to a case where platinum (Pt) is used as a catalyst. For this reason, the use of molybdenum carbide (Mo₂C) as a catalyst is advantageous also in that reduction in hydrogen detection performance caused by CO poisoning can be prevented. A hydrogen gas sensor using molybdenum carbide (Mo₂C) as a catalyst is very useful because such a hydrogen gas sensor can stably detect hydrogen gas even in an environment where the hydrogen gas sensor easily comes into contact with CO, for example, in a fuel-cell vehicle using a flammable gas, such as methane, as a hydrogen source.

According to the present invention, it is possible to reduce the production cost of the hydrogen gas sensor itself, which leads to a reduction in the prices of products using the hydrogen gas sensor. This makes it possible to promote the use of the hydrogen gas sensor in various hydrogen energy systems to ensure safety. For example, the number of hydrogen gas sensors provided in a fuel cell system can be increased or the number of points where the hydrogen gas sensor is provided can be increased to actively take safety measures.

Further, according to the present invention, the catalyst layer made of molybdenum carbide (Mo₂C) is formed by a thin film forming method. This is very advantageous also from the viewpoint, of production. More specifically, according to the present invention, the catalyst layer is formed using a dry thin film forming method, which has the advantages that the process of forming a catalyst layer is simpler as compared to a wet method using a precursor and problems involved in waste liquid treatment can be eliminated.

A general wet method for obtaining molybdenum carbide (Mo₂C) is as follows (see, for example, Japanese Patent Application Laid-open No. 2005-38818). A molybdenum material is chemically combined with oxygen to produce molybdenum oxide (Mo + 1/2O₂ → MoO), and then the molybdenum oxide is subjected to bubbling with a flammable gas such as methane gas to obtain molybdenum carbide Mo₂C.

Unlike such a wet method, a dry thin film forming method to be used in the present invention has the advantage that a catalyst layer can be formed on a solid polymer electrolyte membrane simply by preparing a molybdenum carbide (Mo₂C) sputtering target and directly sputtering the target in a vacuum chamber. Therefore, according to the present invention, it is possible to form a catalyst layer very efficiently without the need to prepare a precursor and a flammable gas and with little regard for production cost. In addition, there is another advantage that the adsorption of CO produced during production to a catalyst can be prevented and therefore the problem of CO poisoning can be completely avoided.

The hydrogen gas sensor according to the fourth embodiment of the present invention using molybdenum carbide (Mo₂C) as a catalyst has been described with reference to a case where the base body composed of a solid polymer electrolyte described with reference to the first embodiment is used as a base body. However, molybdenum carbide (Mo₂C) can be used as a catalyst also when the base body composed of a carbon-containing solid polymer electrolyte described with reference to the second and third embodiments is used as a base body. Also in this case, the same effects can be obtained.

### <Example 4: Structure Using Molybdenum Carbide (Mo₂C) as Catalyst>

### Experiment 4∼1

A hydrogen gas sensor using molybdenum carbide (Mo₂C) as a catalyst (hereinafter, also referred to as a "test sample") and a hydrogen gas sensor using platinum (Pt) as a catalyst (hereinafter, also referred to as a "comparative sample") were prepared, and the amount of change in output caused by contact with hydrogen gas (i.e., transient response characteristics) was measured.

The hydrogen gas sensor prepared as the test sample 1 was produced in the following manner. A Nafion 112 membrane (manufactured by Du Pont) (hereinafter, simply referred to as a "Nafion membrane") was prepared as a solid polymer electrolyte, and was sandwiched between two sheets of filter paper. Then, excess water was removed from both surfaces of the Nafion membrane by pressing an iron heated to 110°C against each of the both surfaces of the Nafion membrane for 3 seconds. Then, one of the surfaces of the Nafion membrane was subjected to sputtering to form a catalyst layer made of molybdenum carbide (Mo₂C) (sputtering conditions: Ar 20 cc/min, RF 300 W, rotation speed of base body 1.6 rpm, sputtering time 90 seconds, thickness of film formed by sputtering 60 nm).

On the other hand, the hydrogen gas sensor prepared as the comparative sample was produced in the following manner. Another Nafion membrane from which water had been removed was prepared, and one of the surfaces of the Nafion membrane was subjected to sputtering under the same conditions as described above to form a catalyst layer made of platinum (Pt).

Then, each of the hydrogen gas sensors prepared as the test sample 1 and the comparative sample was sandwiched between a pair of toroidal electrodes each formed from a SUS plate having a perforated hole at the center thereof, and the electrodes were connected to a digital multimeter (IWATSU VOAC 7411) so that a voltage change could be measured.

In such a state, 40 cc of pure hydrogen gas drawn into a plastic syringe was poured onto the catalyst layer of each of the hydrogen gas sensors sandwiched between the toroidal electrodes. The output voltage of each of the hydrogen gas sensors was measured before and after contact with hydrogen gas to determine the amount of change in output voltage ΔV.

### Experiment 4-2

The influence of CO poisoning on the catalytic activity of the catalyst layer of each of the hydrogen gas sensors prepared as the test sample 1 and the comparative sample was examined in the following manner.

The hydrogen gas sensors prepared as the test sample 1 and the comparative sample were placed in different zippered polyethylene bags, and air was removed from the bags. Then, each of the bags was unzipped, and carbon monoxide gas (99.9%) was introduced thereinto until the bag was inflated by gas pressure so that the surface of the catalyst layer could come into contact with the carbon monoxide gas sufficiently. Then, each of the bags was zipped, and the hydrogen gas sensor was kept hermetically sealed for 30 minutes.

Then, the hydrogen gas sensors prepared as the test sample 1 and the comparative sample were taken out of the bags. Each of the hydrogen gas sensors was sandwiched between electrodes in the same manner as in Experiment 4-1, and the output voltage of each of the hydrogen gas sensors was measured by a digital multimeter before and after contact with hydrogen gas to determine the amount of change in output voltage ΔV.
The results of Experiment 4-1 and Experiment 4-2 are shown in Table 1.

**Table 1**

| Result of Experiment 4-1 (Catalytic Activity under Normal Conditions) | | | |
|---|---|---|---|
| | Output voltage before contact with hydrogen (mV) | Output voltage after contact with Hydrogen (mV) | Amount of change in outputΔV (mV) |
| Test sample 1 | 246 | 226 | 20 |
| Comparative sample | 798 | 550 | 248 |

| Result of Experiment 4-2 (Catalytic activity after CO poisoning) | | | |
|---|---|---|---|
| | Output voltage before contact with hydrogen (mV) | Output voltage after contact with hydrogen (mV) | Amount of change in outputΔV (mV) |
| Test sample 1 | 246 | 225.55 | 20.45 |
| Comparative sample | 798 | 590 | 208 |

### Experiment 4-3

A hydrogen gas sensor using a solid polymer electrolyte membrane having molybdenum carbide (Mo₂C) catalyst layers on both surfaces thereof was prepared, and the amount of change in output caused by contact with hydrogen gas was measured.

A Nafion membrane from which excess water had been removed was prepared in the same manner as in Experiment 4-1, and both surfaces of the Nafion membrane were subjected to sputtering one by one to form catalyst layers made of Mo₂C (sputtering conditions: Ar 20 cc/min, RF 300 W, rotation speed of base body 1.6 rpm, sputtering time 90 seconds, thickness of film formed by sputtering 60 nm). The thus obtained hydrogen gas sensor was defined as a test sample 2.

Then, the test sample 2 was sandwiched between a pair of electrodes in the same manner as in Experiment 4-1, and then a digital multimeter (IWATSU VOAC 7411) was connected to the electrodes. In this state, the test sample 2 was exposed to hydrogen gas to determine the amount of change in output voltage of the test sample 2 before and after contact with hydrogen gas.
The result of Experiment 4-3 is shown in Table 2.

**Table 2**

| Result of Experiment 4-3 | | | |
|---|---|---|---|
| (Catalytic Activity in the Case of Forming Two Catalyst Layers) | | | |
| | Output voltage before contact with hydrogen (mV) | Output voltage after contact with hydrogen (mV) | Amount of change in outputΔV (mV) |
| Test sample 2 | 43.55 | 75 | 31.45 |

Consideration: As can be seen from Table 1, as a result of Experiment 4-1, the amount of change in output ΔV of the test sample 1 using molybdenum carbide (Mo₂C) as a catalyst was as small as 1/20 or less of that of the comparative sample using platinum (Pt) as a catalyst. The difference in output voltage between the test sample 1 and the comparative sample results from the difference in kind of catalyst used. However, in the case of the test sample 1, the absolute value of the amount of change in output ΔV is 20 mV, which is large enough to be used as a detection signal of a hydrogen gas sensor and causes no problems in practical use.

Further, as can be seen from Table 1, as a result of Experiment 4-2, the amount of change in output ΔV of the test sample 1 determined after CO poisoning was not reduced as compared to the amount of change in output ΔV of the test sample 1 under normal conditions determined in Experiment 4-1. This indicates that the performance of the test sample 1 is very stable.

On the other hand, in the case of the comparative sample, the amount of change in output ΔV was reduced by about 40 mV due to CO poisoning of platinum (Pt). This indicates that although platinum (Pt) delivers high catalytic performance under normal conditions and therefore the absolute value of the amount of change in output ΔV is relatively large, the catalytic performance of platinum (Pt) is significantly deteriorated by contact with CO and can vary with a change in environment.

As a result of Experiment 4-1 and Experiment 4-2, it has been confirmed that the hydrogen gas sensor using molybdenum carbide (Mo₂C) as a catalyst has the advantages that it is hardly affected by CO poisoning and shows stable output characteristics and the amount of change in output ΔV is large enough to be used as a detection signal.

Further, as a result of Experiment 4-3, it has been confirmed that the amount of change in output ΔV is increased by forming catalyst layers made of molybdenum carbide (Mo₂C) on both surfaces of the Nafion membrane. This can be attributed to that an increase in the amount of a catalyst led to an increase in the frequency of occurrence of a hydrogen decomposition reaction. Molybdenum carbide Mo₂C is much cheaper than platinum (Pt), which is advantageous in that low cost can be achieved even when the amount of molybdenum carbide (Mo₂C) used as a supported catalyst is increased.

In Experiment 4-3, as described above, the hydrogen gas sensor prepared as the test sample 2 was sandwiched between a pair of toroidal electrodes and the electrodes were connected to a digital multimeter. Therefore, based on the result of Experiment 4-3, the following assumption can be made about how catalytic reaction proceeded in the two catalyst layers of the hydrogen gas sensor prepared as the test sample 2.

The present inventors have already found by another experiment that, as in the case of Experiment 4-3, a hydrogen gas sensor produced by forming platinum (Pt) catalyst layers on both surfaces of a Nafion membrane by sputtering produces a higher output as compared to a case where a platinum (Pt) catalyst layer is formed on one of the surfaces of a Nafion membrane. In this case, the platinum (Pt) catalyst located on the anode side comes into contact with hydrogen and catalyzes the production of protons. On the other hand, it can be supposed that the platinum (Pt) catalyst located on the cathode side reacts with oxygen contained in air and catalyzes the conversion of oxygen to oxygen anions (O₂ + 4e → 2O²⁻). That is, it can be supposed that the reaction at the cathode side is activated by forming catalyst layers on both surfaces of the Nafion membrane so that a water-forming reaction is promoted and therefore the output of the hydrogen gas sensor is increased.

From this consideration, it can be considered that also in the case of Experiment 4-3, the formation of molybdenum carbide (Mo₂C) catalyst layers on both surfaces of the Nafion membrane led to an increase in the output of the test sample 2 on the same principles as described above. That is, it can be considered that, as in the case of the hydrogen gas sensor having platinum (Pt) catalyst layers on both surfaces of a Nafion membrane, the formation of a molybdenum carbide (Mo₂C) catalyst layer also on the cathode side made it possible for the test sample 2 to achieve performance comparable to that of a hydrogen gas sensor using platinum as a catalyst and to exhibit good output characteristics.

The advantages gained by using molybdenum carbide (Mo₂C) as a catalyst have been verified by the above experiments.

The above experiments have been described with reference to a case where the catalyst layer is made of 100% molybdenum carbide (Mo₂C), but another catalyst may be mixed with molybdenum carbide (Mo₂C). Also in this case, the catalytic effect of the catalyst layer is proportional to the amount of molybdenum carbide (Mo₂C) used as an alternative to platinum (Pt).

Examples of such another catalyst to be mixed with molybdenum carbide (Mo₂C) include, in addition to platinum (Pt), gold, silver, iridium, palladium, ruthenium, osmium, nickel, tungsten, molybdenum, manganese, yttrium, vanadium, niobium, titanium, zirconia, and rare-earth metals. In this case, one or more of them can be mixed with molybdenum carbide (Mo₂C).

Further, the above embodiments have been described with reference to a case where the base body constituting the hydrogen gas sensor is composed of a proton conductive solid polymer electrolyte, but the solid polymer electrolyte is not limited to one having proton conductivity. For example, a solid polymer electrolyte having ion conductivity such as an ion exchange membrane may be used. A specific example of such an ion-conductive solid polymer electrolyte includes a NEOSEPTA film manufactured by ASTOM Co., Ltd.

The present invention has been described with reference to various embodiments, but is not limited to these specific embodiments and various changes and modifications can be made without departing from the technical scope of the present invention. Further, two or more of the above embodiments may be combined as long as no contradiction arises.

### INDUSTRIAL APPLICABILITY

The hydrogen gas sensor according to the present invention can be used as a sensor for detecting hydrogen gas leaks in, for example, fuel cell systems for cars and facilities and portable fuel cell systems.

## Claims

1. A hydrogen gas sensor, comprising a detection unit having a base body composed of a solid polymer electrolyte and a catalyst layer provided on one of surfaces of the base body.

2. The hydrogen gas sensor according to claim 1, wherein the base body is composed of a carbon-containing solid polymer electrolyte in which carbon is dispersed.

3. The hydrogen gas sensor according to claim 1 or 2, wherein the base body is composed of a carbon-containing solid polymer electrolyte obtained by dispersing at least one carbon material selected from among carbon nanotubes, fullerene, graphite, nano-carbon, and carbon black in a solid polymer electrolyte.

4. The hydrogen gas sensor according to claim 1, wherein the base body is composed of a carbon-containing solid polymer electrolyte obtained by mixing a solid polymer electrolyte with at least one carbon material selected from a group consisting of carbon black, graphite, nano-carbon, and carbon nanotubes and at least one carbon material selected from a group consisting of fullerene, nano-diamond, and nanoporous carbon.

5. The hydrogen gas sensor according to claim 4, wherein a ratio of the at least one carbon material selected from the group consisting of fullerene, nano-diamond, and nanoporous carbon with respect to total carbon materials contained in the base body is adjusted according to a target range of a hydrogen gas concentration to be detected.

6. The hydrogen gas sensor according to claim 4, wherein a percentage by weight X of the at least one carbon material selected from the group consisting of fullerene, nano-diamond, and nanoporous carbon with respect to total carbon materials contained in the base body is 50 or more but less than 100 (50 ≤ X < 100).

7. The hydrogen gas sensor according to claim 4, wherein a percentage by weight X of the at least one carbon material selected from the group consisting of fullerene, nano-diamond, and nanoporous carbon with respect to total carbon materials contained in the base body is more than 0 but less than 50 (0 < X < 50).

8. The hydrogen gas sensor according to any one of claims 2 to 7, wherein the carbon-containing solid polymer electrolyte is formed by dispersing the carbon material in a solution of a solid polymer electrolyte to obtain a dispersed solution, molding the dispersed solution into a predetermined shape to obtain a molded product, and thermally drying the molded product.

9. A hydrogen gas sensor comprising a detection unit having a base body composed of a carbon-containing solid polymer electrolyte obtained by mixing a solution of a solid polymer electrolyte with two types of carbon materials different in dispersibility in a solvent contained in the solution of the solid polymer electrolyte and a catalyst layer provided on one of surfaces of the base body.

10. The hydrogen gas sensor according to any one of claims 1 to 9, wherein the catalyst layer is made of a metal or an alloy which performs catalytic function on contact with hydrogen gas or an organic metal or an organic substance which exhibits catalytic activity on contact with hydrogen gas.

11. The hydrogen gas sensor according to any one of claims 1 to 9, wherein the catalyst layer is made of a material containing molybdenum carbide which performs catalytic function on contact with hydrogen gas.

12. The hydrogen gas sensor according to claim 10 or 11, wherein the catalyst layer is formed by allowing a catalyst to be supported on one of surfaces of the base body by a thin film forming method selected from among a sputtering method, an ion beam method, a coating method, and a vapor deposition method.

13. The hydrogen gas sensor according to any one of claims 1 to 12, wherein the detection unit is bonded to gas-permeable electrodes.
